(19) Europäisches Patentamt
European Patent Office
Office européen des brevets

(11) EP 4 466 393 B1

(12) EUROPEAN PATENT SPECIFICATION

(45) Date of publication and mention of the grant of the patent:
**25.02.2026 Bulletin 2026/09**

(21) Application number: **23700850.3**

(22) Date of filing: **16.01.2023**

(51) International Patent Classification (IPC):
***C40B 40/06*** *(2006.01)* ***C12Q 1/6869*** *(2018.01)*
***C12Q 1/6806*** *(2018.01)* ***C12N 15/10*** *(2006.01)*

(52) Cooperative Patent Classification (CPC):
(C-Sets available)
**C40B 40/06; C12N 15/1065; C12Q 1/6806** (Cont.)

(86) International application number:
**PCT/EP2023/050812**

(87) International publication number:
**WO 2023/139011 (27.07.2023 Gazette 2023/30)**

(54) **MOLECULAR TAGGING USING POSITION-ORIENTED NUCLEIC ACID ENCRYPTION**

MOLEKULARE MARKIERUNG MITTELS POSITIONSORIENTIERTER NUKLEINSÄUREVERSCHLÜSSELUNG

MARQUAGE MOLÉCULAIRE UTILISANT LE CHIFFREMENT D’ACIDES NUCLÉIQUES ORIENTÉ SUR LA POSITION

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **18.01.2022 EP 22151968**

(43) Date of publication of application:
**27.11.2024 Bulletin 2024/48**

(73) Proprietor: **Universität Potsdam**
**14469 Potsdam (DE)**

(72) Inventors:
- **TAFAZOLI YAZDI, Ali**
**14469 Potsdam (DE)**
- **HOCHREIN, Lena**
**14469 Potsdam (DE)**
- **MÜLLER-RÖBER, Bernd**
**14469 Potsdam (DE)**

(74) Representative: **Hertin und Partner**
**Rechts- und Patentanwälte PartG mbB**
**Kurfürstendamm 63**
**10707 Berlin (DE)**

(56) References cited:
**EP-A1- 3 246 412** **WO-A1-2019/236787**
**CN-A- 111 108 566** **US-A1- 2017 343 545**
**US-A1- 2021 108 194** **US-A1- 2021 121 845**

- **YAO J ET AL: "GENERATION OF EST AND CDNA MICROARRAY RESOURCES FOR THE STUDY OF BOVINE IMMUNOBIOLOGY", ACTA VETERINARIA SCANDINAVICA, SKAND. BLADFORL, DK, vol. 42, no. 3, 1 January 2001 (2001-01-01), pages 391 - 405, XP009040492, ISSN: 0044-605X**

EP 4 466 393 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C12N 15/1065, C12Q 2525/307, C12Q 2563/185; C12Q 1/6806, C12Q 2537/165, C12Q 2563/179, C12Q 2563/185**

## Description

The invention relates to a method for marking a substrate with a unique library of nucleic acid sequence tags comprising: providing a library of nucleic acid sequence tags, comprising at least two different groups of nucleic acid sequence tags that each comprise a plurality of nucleic acid sequence tags, wherein each of said nucleic acid sequence tags comprises a defined nucleic acid motif in at least a first and a second characteristic position, and wherein outside of said at least two characteristic positions each nucleic acid sequence tag comprises a random nucleic acid sequence, wherein the combination of the nucleic acid motifs of the at least first characteristic position and the at least second characteristic position of a nucleic acid sequence tag is characteristic and unique for the group of said nucleic acid sequence tags, and marking the substrate with the unique nucleic acid library by applying the nucleic acid sequence tag library to the substrate. The invention further relates to a packaging article comprising a substrate marked according to the method of the invention and kits for applying and validating a substrate marked according to the present invention.

## BACKGROUND OF THE INVENTION

For products such as paintings, the process of authentication is usually done by experts who look for historical or technical mistakes done by the forger. However, a forged replication (counterfeit) can be so well performed that even experts are misguided, and in the past forged paintings made it into auctions.

For consumable products, such as expensive medicine or kits for biomedical laboratories, authentication is done by controlling the supply chain and using online forms of checking labels.

The shortcoming of such an approach is, first, that it is not always possible to fully control and secure the supply chain, especially if the product is sold internationally. Second, relying solely on labels and barcodes does not provide complete protection of the product from forging. The encryption of the specific pattern of labels is not impossible for forgers and the replication of a label itself is not a difficult task for a professional forger.

Recent advances in using DNA molecules as barcodes have solved some limitations of conventional printed barcodes. In one approach, DNA fragments are used as binary barcodes, which can be identified via DNA sequencing methods using raw data in a relatively fast and easy manner (Doroschak et al., Nat Commun, 2020). This technology avoids some limitations of conventional tagging processes (such as QR codes with printed materials) by enabling tagging objects that are extremely small or flexible. Embodying the barcode at a microscopic level can also help with security issues where the code should not be visible to the naked eye. However, each object still will have one unique barcode. In other words, the DNA barcode according to Doroschak is not secure as the sequence of the barcode can be identified by testing a limited number of DNA molecules. Furthermore, replication and copying of each barcode is possible as each product has only one barcode.

Other early examples of using DNA for personal identification used a personalized set of short tandem repeats (Itakura et al., Int J Inf Secur, 2002; Hashiyada et al., J Exp Med, 2004). However, this approach only uses one defined DNA sequence per product, wherein the sequence can be easily identified and copied with the techniques available today.

WO2019/236787A1 describes an approach for marking products with DNA barcodes. In this approach the sequence and length of the used barcode is not of importance for the encoding, as long as the sequence is known to the provider and customer and can be amplified by PCR. WO2019/236787A1 applies the amount of less than 1 ng of DNA to the product as a security feature against imitation, which is intended to prevent the sequencing and identification of the barcode sequence by forgers. In view of inevitable advance in sequencing technology and improved sequencing sensitivity in the future, the suggested security feature represents a significant shortcoming of this method, as improvements in sequencing technology will likely render it futile.

Other methods of using DNA sequences for encoding messages or data have been suggested, such as in US2005/0059059A1, where DNA is used for stenographic encryption. In this stenographic method messages are encrypted in a DNA sequence that is divided into pieces and interspersed by random nucleotides. The pattern of encoded sequence pieces and random nucleotides is the same among the plurality of DNA fragments encoding a message. This feature renders this method unsuitable for providing protection of a product against imitation by a forger, as by analyzing multiple encoded DNA fragments, the sequence pieces comprising the encoded message could be identified and imitated/copied. Hence, this method is feasible for encoding and transmitting a secret message, but not safe from being copied and imitated by a forger.

US2021/0108194A1 suggests an approach where barcodes are assembled from arrays (building blocks) of short nucleic acid sequences to create libraries of unique barcode sequences. First a binary *in silico* code is generated and translated into a nucleic acid code according to a specific encryption key, which is later used for decryption of the nucleic acid barcodes. One of the shortcomings of this approach is that although a tag library comprises a limited number of different nucleic acid barcode molecules, the code itself is partially encoded by the presence or absence of specific sequences in the barcodes as well as in the randomly chosen sequences, wherefore during decryption a large number of full-length barcode sequences needs to be analyzed, which is time- and cost-intensive and would not confer an economically useful approach for less valuable products that need to be protected from imitation. Another disadvantage of this barcoding approach is that the number of fragments required to find and copy the code is the same as the number of fragments required by the provider or customer to validate the product. Accordingly, although the information of the barcode is encrypted, it is not protected from being copied by a forger. This presents a significant security concern.

By considering the fast progress and development of DNA sequencing and synthesis techniques it becomes clear, that these options are not future proof and will soon be outdated and inefficient.

The authentication of valuable products such as paintings or expensive medicines has always been a challenge. There is a continuous competition between producers of such products and forgers to provide a form of authentication so the buyer can be sure of purchasing the original product.

In view of the shortcomings of existing solutions there exists an urgent need to provide a method that facilitates the reliable identification of valuable products while being safe from forging.

## SUMMARY OF THE INVENTION

The technical object of the present invention is to provide improved means for a reliable, fast and cost-effective way to uniquely label valuable products, while providing a reliable protection from forging. By utilizing the present method, producers or vendors can be certain that the labels they use to mark their valuable products are too difficult or even impossible to forge, also at any time in the future.

The aforementioned problem is solved by the features of the independent claims. Preferred embodiments of the present invention are provided by the dependent claims.

The invention therefore relates to a method of marking a substrate with a unique library of nucleic acid sequence tags comprising: providing a library of nucleic acid sequence tags, comprising at least two different groups of nucleic acid sequence tags that each comprise a plurality of nucleic acid sequence tags, wherein each of said nucleic acid sequence tags comprises a defined nucleic acid motif in at least a first and a second characteristic position, and wherein outside of said at least two characteristic positions each nucleic acid sequence tag comprises a random nucleic acid sequence, wherein the combination of the nucleic acid motifs of the at least first characteristic position and the at least second characteristic position of a nucleic acid sequence tag is characteristic and unique for the group of said nucleic acid sequence tag, and marking the substrate with the unique nucleic acid library by applying the nucleic acid sequence tag library to the substrate.

As is shown in more detail below, the present approach enables the protection of any product from plagiarism that can be marked or tagged according to the invention, such as paintings, artwork, print art, certificates, testaments, consumables worth protecting (e.g., pharmaceuticals), wine bottles, even 3-D printed objects.

Nucleic acids, such as DNA and RNA, comprise four chemical building blocks, called canonical nucleotides. Besides the four canonical nucleotides also chemically modified nucleotides can be present in natural DNA or RNA. In addition, various types of non-naturally occurring nucleotides can be chemically synthesized (synthetic nucleotides). The nucleic acid molecules (tags) used for protection herein consist of a certain number of randomized nucleotides. These nucleotides/tags include coded patterns based on defined relationships between certain nucleotides (characteristic positions). The invention includes in embodiments different designs of nucleic acid libraries (i.e., mixtures of specifically designed nucleic acid molecules, described in detail herein). In one embodiment the invention is meant to protect high-value products with a long lifetime, like paintings. In another embodiment the invention enables protecting high-value consumables which are used in higher throughput. Both embodiments differ in the complexity of the applied pattern (code) and in this way also require different identification tools.

The general concept of the invention is embedding nucleic acid molecules in or on products or packaging materials as a means of authentication proof. It was entirely surprising that the present approach is able to guarantee a high-level protection of diverse products including, but not limited to, valuable goods, artwork, printing and packaging items. Embodiments of the invention include the generation of a pool of diverse nucleotide-based labels encrypting unique codes that are impossible to identify, replicate and forge, if the encryption pattern is not known from the provider of the tag-based label.

To achieve this, in specific embodiments a library of nucleic acid molecules is designed, covering a huge number (typically millions) of different nucleic acid sequences. In embodiments the nucleic acid sequences comprise a certain number of randomized nucleotides, wherein each sequence position contains each type of nucleic acid base that is used within the library in an equal distribution. To guarantee a reliable identification of the nucleic acid libraries in said embodiments, the nucleic acid sequences contain a specific pattern as a code by specifying certain relationships of single nucleotides (e.g. see for specific embodiments of DNA tags Figure 1 and 2) or nucleotide arrays/motifs (e.g. see for specific embodiments of DNA tags Figure 3B). In said embodiments the code, expressed in a simplified manner, is a chosen relationship between the nucleotide motifs in the characteristic positions in a given nucleic acid fragment in between a randomized sequence of nucleotides. Hence, this relationship is based on the distribution of nucleotides in these positions.

In some embodiments within the entirety of tags comprised in a library the sequences of the nucleic acid sequence tags comprise a certain number of randomized nucleotides, wherein certain defined sequence positions or all sequence positions within the random sequence comprise an equal distribution (within the entirety of tags in the library) of only certain types or of a defined selection of nucleic acid bases, which are used within the library. One non-limiting example might be that while a library comprises 4 different nucleic acid bases (e.g. ATCG), only certain types or a defined selection (e.g. the nucleic acid bases T and C) are equally distributed within respective sequence positions of the entirety of tag fragments within library.

In some embodiments defined sequence positions or all sequence positions of the random sequence of a tag comprise some or all types of nucleic acid bases, which are used within the library, at a specific ratio within the entirety of tag fragments of the library. One non-limiting example of such an embodiment might be that a library comprises 4 different nucleic acid bases (e.g. ATCG) and the entirety (the total sum) of tag sequences comprised within said library comprises at sequence position 10 (e.g. when counted in this example from the 5' end of each tag) a ratio of 10:40:25:25 of the nucleic acid bases A:T:C:G or a ratio of 55:20:25:0 of A:T:C:G. In embodiments such specific ratios in defined sequence positions, for example within the random sequence, can add an additional security level for encrypting a code.

In preferred embodiments each sequence position in the random nucleic acid sequence (the sequence outside and/or surrounding the characteristic positions) of the sequence tag should have an equal distribution of the nucleic acid bases used in the library (e.g. the four canonical and other natural nucleic acid bases of DNA, or RNA and/or additional synthetic/non-natural nucleobases), as in a random sequence the chance of being present is the same in each position for each nucleic acid base. It is important that in preferred embodiments by "equal distribution" of all nucleic acid bases, the equal distribution based on the random distribution in each nucleic acid fragment is meant herein and not an exact equal distribution of all nucleobases. This results in a margin of error relative to the number of fragments present in a library and the length of the randomized sequence.

As a non-limiting example, in some embodiments were a nucleic acid fragment or tag has a random sequence comprising 20 bp, and the library comprises 4 different types of nucleic acid bases, the following "equal" distribution may be expected for each nucleotide in each position of the random sequence:

For 10 fragments in a library: 25 $\pm$ 5 %
For 100 fragments in a library: 25 $\pm$ 1.7 %
For 1000 fragments in a library: 25 $\pm$ 0.9 %
For 10000 fragments in a library: 25 $\pm$ 0.7 %
For 100000 fragments in a library: 25 $\pm$ 0.5 %

This example represents one embodiment of calculating the nucleobase composition of a library comprising tags of 20 bp length, wherein the same calculation method of encoding the tag sequences would be used for decoding. This calculation is applied in embodiments of the invention when generating randomized sequences *in silico,* recording all nucleotides in all positions, and calculating the margin of error in comparison to the exact distribution of nucleic acids. This approach can be repeated in embodiments for nucleic acid fragments or tags with different lengths and numbers.

In other words, in embodiments the different nucleic acid bases that are comprised within the library are present in an equal or a nearly equal ratio in each sequence position of the random sequence of the entirety of (all) nucleic acid sequence tags comprised within the library. As in embodiments where every nucleic acid sequence tag in a library has the same length, every tag comprises the same number of sequence positions (e.g. in some embodiments tags of a length of 100 bp/nt translate to 100 positions in the tag sequence), wherein in embodiments each sequence position of the random sequence ideally has the same chance of comprising either of the nucleobases comprised within the library. Hence, in the case of a library comprising an infinite number of tag sequences, the margin of error resulting from the calculated randomization would be close to zero and the calculation of the random sequence of each sequence tag should result in an equal/balanced distribution of the nucleic acid bases, which are used within said library (e.g. in a specific example, four different nucleobases), in each sequence position.

Hence, in embodiments of the invention the entirety of nucleic acid sequence tags comprised within the library comprises in each nucleic acid sequence position each type of nucleic acid base which is comprised within the library at an equal ratio +/- a margin of error relative to the number of total fragments in the entire library and the length of the random sequence of each nucleic acid sequence tag.

Hence, in embodiments of the invention, where the library only comprises the four canonical nucleobases, the nucleic acid bases A, C, T/U and G are present within each nucleic acid sequence position of the entirety of nucleic acid sequence tags comprised within a library each at 25% +/- a margin of error relative to the number of total fragments in the entire library and the length of the randomized sequence of each nucleic acid sequence tag. In other words, in embodiments the entirety of nucleic acid sequence tags comprised within a library comprises in each nucleic acid sequence position the nucleic acid bases A, C, T/U and G at a percentage of each 25% +/- a margin of error relative to the number of total fragments in the entire library and the length of the total sequence, preferably of the random sequence of each nucleic acid sequence tag. Accordingly, in embodiments the nucleic acid bases A, C, T/U and G are each present at a percentage of 25% +/- a margin of error relative to the number of total fragments in the entire library and the length of the random sequence of each nucleic acid sequence tag, within each nucleic acid sequence position of the entirety of nucleic acid sequence tags comprised within the library.

Hence, a surprising key benefit of the present method is the implementation of a simple, yet secure, pattern in a nucleic acid sequence. The core element of the pattern is having a defined relationship between the characteristic positions among all other random positions that do not follow any pattern.

The margin of error according to the present method might be in specific embodiments 0.01%, 0.05%, 0.1%, 0.25%, 0.5%, 0.75%, 1%, 2%, 3%, 4%, 5%, 6%, 7%, 8%, 9%,10%, 15%, 20%, 25% or 30%.

In embodiments a random nucleic acid sequence of the tag might have a length of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 45, 46, 47, 48, 49, 50, 51, 52, 53, 54, 55, 56, 57, 58, 59, 60, 65, 70, 75, 80, 85, 90, 95, 100, 110, 115, 120, 125, 130, 135, 140, 145, 150, 155, 160, 165, 170, 175, 180, 185, 190, 195, 200, 210, 215, 220, 225, 230, 235, 240, 245, 250, 255, 260, 265, 270, 275, 280, 285, 290, 295, 300, 350, 400, 450, 500, 550, 600, 650, 700, 750, 800, 850, 900 or 1000 base pairs. In preferred embodiments the nucleic acid sequence tag has a length between 10 and 1000 bp, even more preferably of 10-500 bp.

In some embodiments all nucleic acid sequence tags comprised within a library have the same length. In other embodiments the nucleic acid sequence tags comprised within a library are of different lengths. In embodiments where the nucleic acid sequence tags are of different length the position of the characteristic positions within the nucleic acid sequence of the tag may in some embodiments be set/determined from the center of the nucleic acid sequence tags, or may be set/determined either from the start or from the end of the tag. In other words, the "starting point" for the setting/determination of the characteristic positions would either be the center, the start or the end of a tag sequence. In such embodiments the "starting point" from which the characteristic position would be set/determined would be the same for at least each group of tags.

In preferred embodiments all sequence positions (random or characteristic) of the entirety of nucleic acid sequence tags comprised within the library should have an equal or nearly equal distribution of each type of nucleic acid base used within the sequences of the library (e.g. in some embodiments of libraries that only comprise the canonical nucleobases: 25% A, 25% T/U, 25%C, 25%G, each +/- a margin of error). This makes it hard to identify the characteristic nucleic acid bases as the forger needs to check the relationship between an unknown number of different bases. Due to said about equal distribution of nucleic acid bases in each sequence position of the entirety of nucleic acid sequences comprised within the library it is difficult for a forger to determine the location and number of the characteristic positions and hence to imitate the characteristic pattern encoded therein.

Hence, in embodiments of the present method the aim is to also have the same/equal distribution of nucleotides in the characterizing positions as in the random positions. So, by analyzing random and characterizing positions, one should not be able to find any difference in the distribution of nucleotides/nucleobases. Hence, as each sequence position in the library has the same equal distribution of nucleotide bases, a forger would not be able to identify where the characterizing positions are located within a tag sequence and how the code pattern looks like, as each tag sequence appears completely random to someone not knowing the pattern.

In embodiments of the invention at least 80%, preferably at least 90%, of the nucleic acid sequence tag molecules within the library comprise a nucleic acid sequence that is unique within the entire library. In preferred embodiments of the invention at least 95%, even more preferably at least 99%, of the nucleic acid sequence tag molecules within the library comprise a nucleic acid sequence that is unique within the entire library.

In embodiments of the invention wherein each characteristic position comprises a motif that is only one nucleotide in length, each type of nucleobase used within the library is present in an equal ratio within each nucleic acid sequence position of the at least first and the at least second characteristic position of the entirety of nucleic acid sequence tags comprised within the library, as a) the nucleobases in the at least first and second characteristic position of the nucleic acid sequence tags are selected accordingly and/or the groups of nucleic acid sequence tags are comprised within the library at a specific ratio, or b) a sufficient amount of additional nucleic acid sequence tags which comprise selected nucleobases in the at least first and second characteristic position is comprised within the library.

Hence, in embodiments where each characteristic position comprises a motif that is only one nucleotide in length, each type of nucleic acid base which is comprised within the library is present at an equal ratio within each of the at least first and the at least second characteristic position of the entirety of nucleic acid sequence tags comprised within the library, as a) the nucleic acid bases in the at least first and the at least second characteristic position of the nucleic acid sequence tags are selected accordingly and/or the groups of nucleic acid sequence tags are comprised within the library at a specific ratio, or b) a sufficient amount of additional nucleic acid sequence tags which comprise selected nucleobase in the at least first and second characteristic position is comprised within the library.

Non-limiting examples of specific embodiments of this approach are depicted in Figures 1 and 2.

The encryption of information according to embodiments of the present invention is achieved by the combined features of tags comprising a random nucleotide sequence, a (nearly) balanced distribution of nucleotide bases within the sequences of the tag library and the motifs and location of the characteristic positions. Said features achieve several advantageous and surprising effects such that for each tag molecule a unique sequence of nucleotides is generated, while only a certain degree of information is actually encoded, namely by the characteristic positions. This enables the generation of an unexpectedly high number of unique tag sequences, even in embodiments of tags of a short length, such as 10-20 bp, for each unique pattern of characteristic positions. Hence a huge number of products can be uniquely and safely marked and protected from forgery. The high security from being decrypted and imitated by forgers provided by the tags generated according to the present invention hence results in a significantly improved protection of tagged products from forgery. Another advantageous effect is that the generation of unique tag sequences according to the invention does not require time intensive barcode construction and as only a small amount of library needs to be applied to a substrate the present method is also cost-effective in terms of oligonucleotide synthesis. Contrary to many prior art methods, the detection of the information encoded by the tags according to the present method does not require the sequencing of a high number of nucleic acid fragments, as the specific pattern of the characteristic positions can be validated from a small number of tag fragments, as shown in the Examples herein.

In general, a possibility to forge a product covered with regular state of the art DNA tags would be to identify a small number of included sequences and copy them. This small fraction could cover all unique sequences in a barcode if the variety of fragments are not big enough. However, in the case of the present method the variety of the tag sequences comprised by the library is high enough, such that copying a small proportion of unique sequences from a library will result in a change of the proportions of unique sequences in the resulting forged tag library.

The following calculation shows this effect in an example embodiment that can also be used as a source of validation in some embodiments.

In a non-limiting example of a design where X is the number of unique sequences that are comprised within a group tags and Y is the number of sequences that are analyzed during validation, the probability (P) that all analyzed sequences are unique is calculated by:

$$P = (X/X) * (X-1/X) * (X-2/X) \ldots * (X-(Y-1)/X) = X! /((X-Y)! *(X^Y))$$

The following example calculates P for scenarios where each group of tags comprises either 10 or 20 unique sequences, and wherein 5 sequences are analyzed:

P for a group of tags with 10 unique sequences: 10!/5!*10^5 = 0.30

P for a group of tags with 20 unique sequences: 20!/15!*20^5 = 0.58

Accordingly, the probability of extracting unique sequences is higher if the number of unique sequences within a group of tags is higher. In embodiments this probability can be used to validate the uniqueness of sequences within the tag library. The number of sequences required to be analyzed for this validation approach is dependent on the specific design of an embodiment and its applications. This validation approach can be used in embodiments when a large number of unique sequences are comprised by a group of tags. The present approach also provides a highly cost-effective method to provide an applicable tag-library of unique sequences. In embodiments where the extraction efficiency of a tag library from an original product by a forger does not equal 100% also the copying process cannot be complete, wherefore a forged library can easily be distinguished from a legitimate library, as such a forged library would lack complexity. In addition, in preferred embodiments the library generated according to the invention comprises only unique sequences, or only few duplicates/a small percentage of duplicates (as the generation of a few duplicates due to errors in nucleic acid synthesis or *in-silico* sequence calculation cannot always be excluded). In embodiments this (near) uniqueness of the tags comprised within a valid tag library provides additional protection, in the case where a forger sequences and copies only a fraction of the original library, that was applied to a substrate. To achieve a sufficient concentration of a library to be applied to forged product, such a forged library would need to comprise numerous duplicate sequences and could easily be identified as a forged library.

The same applies to the labelling of multiple batches of products. The complexity and hence the number of unique sequences within a library according to the present method can in embodiments be easily customized by selection of the tag length and the number of characterizing positions. Hence, in embodiments where multiple product batches are labelled according to the present method, they would not comprise duplicate tag sequences, while still having the same "code"/pattern encoded by the characterizing positions. Accordingly, if a high number of duplicate tag sequences are detected during validation between different batches, or even entirely duplicate libraries, it can be assumed that the product is falsified and was labelled with a copied tag library illegally obtained from a legitimate product.

In summary, due to the combination of the characterizing positions with the random sequence, the identification pattern/code encoded according to the present invention is basically invisible and impossible to identify by a forger. Accordingly, as described herein before the present approach not only facilitates the validation via the specific design/code encoded within the characterizing positions but also enables the validation of a product through the analysis of the uniqueness of tag sequences, e.g. within a single library applied to a product or between batches of a product. Such an effect could not be achieved using prior art barcoding methods, such as US2021/0108194A1.

In embodiments of the invention only specific combinations of nucleic acid motifs in the at least first and the at least second characteristic position are used within the nucleic acid sequence tags. This means that, for example, from all possible combinations of two motifs only a selected portion is used within the library. This has the advantage that through exclusion of combinations the security from forgery of a library can further be improved. Non-limiting examples of such embodiments are depicted in Figure 4.

In specific embodiments a library consists of DNA sequences with a length of each 100 nucleotides with a pattern consisting of a defined relationship between two characteristic positions with a single nucleotide motif, said library might comprise two different groups of tags at an equal ratio: group 1: if the first characteristic position is nucleotide A or T, the second characteristic position should be C or G. All nucleotides besides those at the first and second characteristic position are randomized, namely chosen randomly. Group 2: if the first characteristic position is nucleotide C or G, the second characteristic position should be A or T. All nucleotides besides those at the first and second characteristic position are randomized. In this specific embodiment, each characteristic position (first and second) will have the same distribution of nucleic acids as the randomized positions. A non-limiting example of this embodiment is depicted in Figure 1. The coded pattern can only be detected when knowing the sequence position of the characterizing positions, followed by checking the distribution of base pairs between two characteristic positions.

In embodiments of the invention nucleic acid sequence tag molecules or nucleic acid sequence tags are present as linear nucleic acid molecules.

In some embodiments of the method according to the invention each nucleic acid sequence tag is present as a circular nucleic acid molecule or is comprised inside a circular nucleic acid molecule. In some embodiments the nucleic acid sequence tag molecules are circularized or 3'-5'end ligated with themselves to form a circular molecule, which preferably only comprises the tag sequence.

In other embodiments the circular nucleic acid molecule comprises the tag sequence plus a sequence comprising a ligation adaptor, a ligation site or sticky ends, which were used for circularization. Methods for nucleic acid circularization are known to the person skilled in the art.

In other embodiments the nucleic acid tag sequences comprised within the library are cloned, embedded or comprised inside a circular DNA molecule, such as a plasmid or vector, before they are applied to the object to be marked (see e.g. Figure 1 for one specific embodiment). In some embodiments the tag molecules can be cloned or inserted inside a circular nucleic acid molecule before they are added to the library. In some embodiments the nucleic acid tag sequences are cloned or inserted inside circular nucleic acid molecules after being added or pooled into the library. Accordingly, in such embodiments the entire library comprising the nucleic acid sequence tag molecules is subjected to a cloning step, wherein the single tag molecules are each cloned inside circular nucleic acid molecules, such as e.g. plasmids or vectors. After the cloning steps in the certain embodiments' optional purification, washing and/or enrichment steps might be performed, to select for/enrich the tag-comprising circular nucleic acids.

The provision of the nucleic acid sequence tags as circular nucleic acids can improve the stability of the nucleic acid molecules, e.g. from nuclease digestion or other hazards. Hence, in some embodiments where a tag library with a long lifetime is desired the nucleic acid sequence tags may be provided as DNA sequence and optionally may be provided as circularized nucleic acids. A circularized nucleic acid may in embodiments be obtained either through circularization of a nucleic acid sequence tag molecule or by cloning a nucleic acid sequence tag molecule into a DNA vector or plasmid, or by providing a nucleic acid sequence tag molecule as chirally inverted L-DNA, which is understood to have an improved resistance to biodegradation, compared to the naturally occurring D-conformation of DNA (Fan et al., Nat Biotechnol, 2021).

Accordingly, in certain embodiments the nucleic acid sequence tags within a library are present as linear nucleic acid molecules. In other embodiments the nucleic acid sequence tags within a library are present as circular nucleic acid molecules. In certain embodiments a combination of circular and linear nucleic acid molecules might be present within a library.

Nucleic acid molecules can be double stranded or single stranded molecules. In some embodiments the nucleic acid sequence tags within a library are double stranded nucleic acid molecules. In other embodiments the nucleic acid sequence tags within a library are single stranded nucleic acid molecules. In certain embodiments a combination of single and double stranded nucleic acid sequence tag molecules might be present within a library.

In embodiments where the product to be protected is or comprises a printed product or packaging it may be marked by embedding the tag library comprising the nucleic acid sequence tags in the paper/cardboard or in the ink of the printed product or packaging. In embodiments the customer, vendor or distributor can take for validation a sample from the marked item (from where the tag library was applied) analyze it or request the provider or distributor to analyze the sample, e.g. by sequencing or PCR, for the correct nucleic acid sequence tag patterns. In the case where a customer can analyze the library himself, simply the key or code-pattern of the library can be requested from the producer or provider. The concept applied in these embodiments allows an adaption of the complexity of the integrated tag library with respect to the producers', distributors' and/or customers' needs and possibilities. Furthermore, the flexibility of the design allows for easy adaptation of the encoded tag pattern (which might e.g. be relevant in embodiments where different batches or different levels of value of a product are to be protected).

In summary, to the before stated problem, the present method provides a solution that allows a way of authentication that ensures that a forger can never replicate the original product, regardless of his possibilities and skills. The present invention provides in embodiments the option to easily enlarge the complexity of the nucleic acid-embedded code to protect any product of interest. A forger not only needs to isolate and identify the nucleic acid pattern used in the method according to the present invention but would also need to synthesize the designed nucleic acid sequence tags and apply it onto the false product. This will make it much more difficult or even impossible to fake the respective product.

The same advantages apply to consumable products, as the present method facilitates the provision of a secure authentication method for each product which is not possible with conventional state of the art barcoding approaches.

The present method also has the capacity to make a lasting contribution to the general art of tagging/marking and identifying valuable goods, as in embodiments the nucleic acid sequence tag structure according to the invention is, especially if DNA is used, stable for hundreds of years as it is technically just not possible to identify the code encoded within the library of nucleic acid sequence tag molecules, irrespective of the potential method used, even when considering future developments in nucleic acid sequencing or analysis methods.

One of the major goals of the present method is a high security level of the nucleic acid codes also at any given time in the future, regardless of the state of the sequencing technology. To achieve this, the complexity of the tag/barcode design in embodiments of the invention may be low enough to allow the clear identification of nucleic acid sequences for those knowing the code, but high enough to exclude an identification by a forger not knowing the code. In embodiments several layers of complexity can be implemented, to achieve the highest possible security based on the technological state-of-the-art at a given time.

In another preferred embodiment the complexity of the design can be tuned by changing the length of the nucleic acid molecule and the number and position of selected nucleic acid bases included in the coded pattern. This gives the possibility to provide a great number of unique tags. This is, for example, important in embodiments where different batches of a product need to be marked. Second, the complexity of the coded patterns can simply be adjusted to the customers' needs using the core principle of the design (relational distribution of nucleic acids). Increasing the number of positions in the pattern in embodiments will increase the complexity of the design. Hence, finding and replicating the design pattern by a forger would be even more difficult.

In embodiments a nucleic acid sequence tag might have a length of more than 1 bp, or 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 45, 46, 47, 48, 49, 50, 51, 52, 53, 54, 55, 56, 57, 58, 59, 60, 65, 70, 75, 80, 85, 90, 95, 100, 110, 115, 120, 125, 130, 135, 140, 145, 150, 155, 160, 165, 170, 175, 180, 185, 190, 195, 200, 210, 215, 220, 225, 230, 235, 240, 245, 250, 255, 260, 265, 270, 275, 280, 285, 290, 295, 300, 350, 400, 450, 500, 550, 600, 650, 700, 750, 800, 850, 900 or 1000 base pairs. In preferred embodiments the nucleic acid sequence tag has a length between 10 and 1000 bp, even more preferably of 10-500 bp.

In one specific embodiment, increasing the number of characteristic positions from two to three will improve the security around ten times, whereas the difficulty of validating the design will increase less than three times. The number of characteristic positions (and patterns as a result) is theoretically limited by the length of the nucleic acid sequence. Hence, the tag design according to the invention provides noticeable flexibility for the producer and consumer based on the product quantity and required security.

In embodiments the nucleic acid sequence tag comprises more than1 characteristic position, or 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 45, 46, 47, 48, 49, 50, 75 or even 100 characteristic positions. In preferred embodiments the nucleic acid sequence tag comprises 2-25 characteristic positions, in some embodiments 2-10 or 2-5 characteristic positions.

The inventors established different embodiments of the designs for the nucleic acid-coded pattern. In the context of the present invention, any embodiment described herein might be used to mark or tag any substrate or product of choice. The herein mentioned embodiments are not meant to limit certain embodiments of the method according to the invention to certain products, they are merely presented as examples.

In preferred embodiments a characteristic position is defined by a motif comprising only a single nucleotide. In some embodiments this design can be used for high-value and long-lasting products, like paintings, artwork, antiques or testaments. The applied validation method relies in preferred embodiments on nucleic acid sequencing, which is more time- and cost-intensive, but allows for reliable identification of nucleic acid molecules. In other embodiments this specific approach may also be used for tagging any desired product, even low-value products can be marked according to this approach.

In further preferred embodiments an array of nucleotides (at least two consecutive nucleotides) at a characteristic position is used among randomized positions. In some embodiments this can be used to protect consumables like pharmaceutical products. Due to the relatively short lifetime of products in this category, a fast, cheap and easy validation test might be desirable, which is possible to be performed faster, with less effort and in higher throughput.

In embodiments the nucleic acid motif in a characteristic position comprises 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 125, 150, 200, 250, 300, 350, 400 or 500consecutive nucleotides. In some embodiments the nucleic acid motif in a characteristic position comprises between 1 and 15 consecutive nucleotides. In some embodiments the nucleic acid motif in a characteristic position comprises between 1 and 50 consecutive nucleotides. In some embodiments the nucleic acid motif in a characteristic position comprises between 1 and 10 consecutive nucleotides. In some embodiments the nucleic acid motif in a characteristic position comprises 8 consecutive nucleotides. In some embodiments the nucleic acid motif in a characteristic position comprises 1 nucleotide.

Accordingly, in embodiments of the invention the nucleic acid motif in the at least first and the at least second characteristic position each comprises a nucleotide sequence of at least two consecutive nucleotides. In specific embodiments the at least first and the at least second characteristic position each comprise a motif comprising a nucleotide sequence of at least three, four, five, six, seven, eight, nine, ten, eleven, twelve, thirteen, fourteen, fifteen, etc. consecutive nucleotides.

In embodiments using an array of nucleotides in the characteristic position enables the use of molecular techniques such as sequence specific restriction assays, immuno-assays, assays using labelled hybridization probes or CRISPR/Cas-based assays for a fast and accurate validation test using restriction. SHERLOCK (Kellner at al., Nat Protoc, 2019) and DETECTR (Chen et al., Science, 2018, Apr 27;360(6387):436-439) are examples of such techniques that have been developed for using CRISPR/Cas for rapid nucleic acid sequence detection.

In embodiments the library may also be validated using a dCas9-based assay incorporating sgRNAs specifically binding to the correct motif(s) (in the characteristic positions). If the target sequence motif is available, the sgRNA binds the sequence which will result, for example, in an enzymatically catalyzed color shift as a read-out. In embodiments for this validation assay, already published detection methods can be used (Chen et al., Science, 2018, Kellner et al., Nature Protocols, 2019). Such test kits could be applied in a rapid and cost-effective manner without specific knowledge.

Any other method for detection and identification of short nucleotide arrays is possible and the person skilled in the art is aware of such techniques for nucleic acid analysis. Also, hybridization or PCR assays with primers or probes provided in a separate "validation" kit according to the invention from a provider or producer of the tagged product can be used in embodiments.

However, the complexity of embodiments allying an array of nucleotides as a characteristic position might not be as high as in embodiments using single nucleotides in characteristic positions. In some embodiments the restriction-based detection, e.g. by CRISPR /Cas9-based assays, can be used in combination with an online verification process (e.g. for a non-limiting Example see Fig. 3).

In embodiments one application of the tagging-method according to the invention can be a product package that comprises one or more, in a specific example three or more spots, each of which comprises a different nucleic acid sequence tag library or a different group of nucleic acid sequence tags. Each spot would be tested to give a negative or positive result. The combination of these results could be used for online authentication. So, to forge products marked according to this specific embodiment, not only the forger should mimic the test positive and negative results, also the same result pattern would have to be mimicked as well. This might discourage potential forgers, as this might not be possible or feasible for most forgers. In addition, the validation assay might be performed using a kit according to the present invention which might be provided or along or separately with the valuable product marked/tagged according to the invention, to add an additional level of security.

In embodiments of the invention the amount of library that is applied to the substrate comprises 10 to 10^15 times, preferably 100 to 10^14 times, the number of nucleic acid sequence tag molecules that have to be analyzed (A) from the applied library for a specific certainty of correct validation (V), wherein the specific certainty of correct validation (V) is calculated according to the formula:

$$V = (1- (R^A))*100,$$

wherein R is the ratio of the number of unique combinations of nucleic acid motifs at the at least first and second characteristic positions comprised within the library over the number of possible unique combinations of motifs.

In embodiments of the invention the certainty of correct validation (V) is determined by the number of nucleic acid sequence tag molecules that are analyzed (A) from the applied library wherein the specific certainty of correct validation (V) is calculated according to the formula:

$$V = (1- (R^A))*100,$$

wherein R is the ratio of the number of unique combinations of nucleic acid motifs at the at least first and second characteristic positions comprised within the library over the number of possible unique combinations of motifs.

In another embodiment, the desired certainty of validation may be 50%, 55%, 60%, 65%, 70%, 75%, 80%, 85%, 90%, 91%, 92%, 94%, 96%, 97%, 98%, 99%, 99.5% or 99.9%. In a preferred embodiment the confidence interval is between 95-99.9%. In some embodiments, the desired certainty of validation may be at least 50%, at least 75%, at least 80%, at least 85%, at least 90%, at least 95% or at least 98%.

In embodiments the number of tag molecules applied to a substrate might be based on the limitation of the present sequencing technology.

The current limitation of PCR-free sequencing is 1 ng of nucleic acid (as it is mentioned also in patent WO 2019/236787 A1). This equals about 10^13 nucleic acid molecules of 100 bp length. Considering this number, even though in certain embodiments at least 10 nucleic acid molecules are required for reliable validation, in some embodiments at least 1 ng of nucleic acid sequence tag molecules/library must be applied to the substrate. Accordingly, in some embodiments relating to the present accuracy and sensitivity of sequencing technology at least 10^12 times the number of nucleic acid molecules necessary for reliable validation (=10 molecules) is applied. Furthermore, in certain embodiments the efficiency of nucleic acid extraction can also affect this number. For example, in embodiments where 1 ng of nucleic acid should be extracted about 10 to 100 ng of nucleic acid sequence tags might need to be applied, hence the number of nucleic acid sequence tags applied in these embodiments would be between 10^12 to 10^14 times the validation required amount. In preferred embodiments this number applied to the substrate will still be less than the amount required for finding and forging the tag design and will decrease as the technology of sequencing advances.

Accordingly, one of the most important aspects of the present invention is the advantage of having a flexible pattern for authentication. In embodiments during the process of authentication of any given library, the number of samples required for validating a library of nucleic acid molecules (for those knowing the coded pattern) is X times lower than the number of samples required to identify ("crack") the pattern (for those not knowing the coded pattern). X is dependent on the number of characteristic positions (randomized and defined) and the selected relationship/combination. If the number of characteristic positions increases, the minimum number of samples required to identify the pattern and X will increase. By limiting the number of nucleic acid sequences provided in a given library applied on a certain product in certain embodiments, it can be guaranteed that the coded pattern cannot be decoded by anyone not knowing the code. In embodiments this is due to the fact that the number of applied nucleic acid molecules (and, hence, sequences) is lower than the minimum number of nucleic acid sequence tag fragments necessary for successful identification by a forger not knowing the code. In this way, in embodiments it can be ensured to have enough tag molecules for validating the known pattern (by provider or customer) but not enough tag molecules to identify and decode an unknown pattern (by forger).

In one embodiment the maximum number of nucleic acid sequence tag fragments/molecules within a library that is applied to a substrate may be 1000, 5000, 10,000, 20,000, 30,000, 50,000, 100,000, 150,000, 250,000, 500,000, 1 million, 1.5 million, 2 million, 5 million, 10 million, 50 million, 100 million, 10^8, 10^9, 10^10, 10^11, 10^12, 10^13, 10^14, 10^15, 10^16, 10^17, 10^18, 10^20, between 10^2 and 10^15, between 10^5 and 10^16, between 10^10 and 10^14, between 10^5 and 10^14 nucleic acid molecules or any number or range in between. In some embodiments between 10^5 and 10^14 nucleic acid sequence tag molecules are be applied to the substrate.

In embodiments the amount of nucleic acid library applied to a substrate may be between 0.1 and 100,000 ng, between 10 and 10,000 ng, between 100 and 1000 ng, between 1 and 1,000 ng, between 1 and 500 ng, between 1 and 100 ng, between 1 and 10 ng, 0.1 ng, 0.5 ng, 1 ng, 2.5 ng, 5 ng, 7.5 ng, 10 ng, 50 ng, 100 ng, 250 ng, 500 ng, 1000 ng, 1500 ng, 5000 ng, or 10,000 ng. In some embodiments 0.5 to 5 ng may be applied.

In embodiments of the invention a substrate is selected from the group of paper, cardboard, canvas, ceramic, cotton, wool, leather, nylon, plastic, vinyl, stone, metal, glass, liquid, a pharmaceutical, a packaging article, sculptures, paintings, artwork, computers, computer peripheral devices, printers, microchips, televisions, sound systems, furniture, appliances, antiques, clothing, handbags, shoes, sunglasses, cameras, automobiles, bicycles, motorcycles, luggage, 3-D printed objects or collectibles.

In a preferred embodiment the invention relates to a method of marking a substrate with a nucleic acid sequence tag library, wherein the nucleic acid sequence tags or nucleic acids are selected from the group of DNA, gDNA, RNA, gRNA, mRNA and cDNA, or any combination thereof. In preferred embodiments the nucleic acids are DNA.

In embodiments of the present invention the library of nucleic acid sequence tags that is applied to the substrate is configured to be analyzed a) by extracting nucleic acid sequence tags of the library from the substrate and analyzing the nucleic acid sequence tags, or b) by analyzing nucleic acid sequence tags of the library directly on the substrate.

In specific embodiments analyzing the nucleic acid sequence tags comprises performing one or more of the analysis methods selected from the group comprising nucleic acid sequencing, nucleic acid restriction assays, hybridization assays, immunoassays, CRISPR-Cas assays, molecular beacon assays, PCR, digital PCR, qPCR and real-time PCR.

In another aspect the present invention further relates to a packaging article, wherein the packaging article comprises a substrate that has been marked according to the method of the invention. In some embodiments of the packaging article the article comprises 1 or more, for example 1, 2, 3, 4, 5, 6, 7, 8, 9, 10, 12, 13, 14, 15, 16, 17, 18, 19, 20 or 25 substrates that were each labelled with a different or the same tag library or with different or the same groups of sequence tags.

In another aspect the present invention relates to a kit for carrying out the method according to the invention.

In one embodiment the kit comprises one or more libraries according to the invention comprising a plurality of nucleic acid tag sequences according to the invention, and optionally means for applying the library according to the invention to the product. In another embodiment of the kit, the kit further comprises additional means for protecting, covering and/or sealing the applied library on the product.

In embodiments the kit comprises means for validating the library according to the invention. Such means may comprise means for extracting, detecting and/or analyzing nucleic acids and/or optionally information on the pattern encoded within the library.

In one aspect the invention relates to a kit for analyzing a library of nucleic acid sequence tags that has been applied to a substrate according to the method of the invention comprising means for performing at least one of the analysis steps selected from the group comprising nucleic acid sequencing, nucleic acid restriction assays, hybridization assays, immunoassays, CRISPR-Cas assays, molecular beacon assays, PCR, digital PCR, qPCR and real-time PCR, and optionally means for nucleic acid extraction.

The invention also relates to a nucleic acid sequence tag generated according to the method of the invention. The invention further relates to a nucleic acid sequence tag for use in the tagging method of the invention.

The invention further relates to a nucleic acid sequence tag library comprising a plurality of nucleic acid sequence tags according to the present invention. The invention also relates to a nucleic acid sequence tag library for use in the tagging method of the invention.

In some embodiments of the library according to the invention, each nucleic acid sequence tag within the plurality of nucleic acid sequence tags is comprised within or cloned inside a circular nucleic acid molecule, wherein the circular nucleic acid molecule is preferably a vector or plasmid.

Embodiments of the invention are not limited to tags comprised of nucleic acids. The present method may in embodiments also be performed using tags comprised of any organic building block, biopolymer or polymer, such as amino acids, sugars etcetera. Any kind of chemical building block with a diversity of $n \geq 2$ may be employed for establishing the code encrypted in the tags according to the invention, as the coding principle is entirely independent of its chemical implementation. Accordingly, embodiments of the present method also relate to a tagging method using tags comprised of a chemical or biological polymer that can comprise and encode at least a first and a second characteristic position surrounded by a random sequence.

The embodiments described herein for one aspect of the invention may also be embodiments of any one of the other aspects of the present invention. Accordingly, embodiments described for the method according to the invention may also be embodiments of the kit or the nucleic acid sequence tag library disclosed herein. In addition, any embodiment described herein, may also comprise features of any other embodiment of the invention.

## DETAILED DESCRIPTION OF THE INVENTION

All cited documents of the patent and non-patent literature are hereby incorporated by reference in their entirety.

The present invention is directed to a method for marking a substrate with a unique library of nucleic acid sequence tags, wherein the nucleic acid sequence tags comprise at least two characteristic positions comprising nucleic acid motifs and a surrounding random nucleic acid sequence, that is preferably unique to each nucleic acid sequence tag molecule.

In general, "nucleic acids" are biopolymers, or large biomolecules composed of nucleotides. Herein, nucleic acid may preferably refer to DNA (deoxyribonucleic acid), gDNA (genomic deoxyribonucleic acid), RNA (ribonucleic acid), gRNA (genomic ribonucleic acid), mRNA (messenger ribonucleic acid), cDNA (complementary deoxyribonucleic acid synthesized from RNA template), synthetic DNA or RNA, or DNA or RNA comprising synthetic nucleotides, or any combination thereof. In a preferred embodiment a nucleic acid or nucleic acid sequence herein is DNA.

"Nucleotides" are organic molecules composed of three subunit molecules: a nucleobase, a five-carbon sugar (ribose or deoxyribose), and a phosphate group consisting of one to three phosphates. "Nucleobases" are nitrogen-containing biological compounds and can also be referred to as "nucleic acid bases" or "bases". Accordingly, said terms might be used herein interchangeably. Nucleobases comprise in case of the primary or canonical nucleobases of DNA guanine (G), adenine (A), cytosine (c) and thymine (T) and uracil (U) -instead of thymine- in RNA. In addition, besides the primary or canonical nucleobases of DNA and RNA any other synthetic and/or naturally occurring nucleobase, and/or any (chemically) modified nucleobase (e.g. hm5C, (5-hydroxymethylcytidine), m5C (5-methylcytidine), m6A, (N6-methyladenosin), 5fC (5-formylcytosine), 5caC (5-carboxylcytosine) etc.) can be used in the context of the present invention. The skilled person is aware of all possible chemical modifications and synthetic and/or naturally occurring nucleobases (e.g. McCown et al., 2020, WIREs RNA; Sood et al., 2019, J Cheminform; https://dnamod.hoffmanlab.org/). Herein (chemically) different nucleobases (e.g. A, G, C, T, hm5C, m5C, m6A, 5fC, 5caC or any other nucleobase) are considered different "types" of nucleobases or nucleic acid bases. When referring to "different types of" or "different" nucleotides, nucleic acid bases or nucleobases herein, generally nucleotides comprising different types of nucleobases or nucleic acid bases (e.g. A, G, C, T, hm5C, m5C, m6A or any other nucleobase) are meant.

Nucleic acids or nucleic acid molecules can be present as single stranded (ss) or double stranded (ds) molecules, e.g. double stranded or single stranded DNA or RNA (ssRNA, dsRNA, ssDNA or dsDNA). Accordingly, in embodiments of the present invention the nucleic acid sequence tag molecules or nucleic acid sequence tags can be present as single stranded or as double stranded molecules.

"Nucleic acid sequences" refer herein to a consecutive array of nucleotides, wherein the nucleotides are distinguished by their nucleobases or nucleic acid bases. A nucleic acid sequence may herein also refer to the sequence of consecutive letters (in embodiments comprising only the naturally occurring canonical nucleobases comprised of G, A, C and T or U) that represent the actual sequence of consecutive nucleic acids in a strand of DNA or RNA. In embodiments of the invention, to increase the complexity of the code encrypted within a tag sequence, in addition or instead of natural occurring canonical nucleobases A, G, C and T or U, also any kind of synthetic nucleobases or any other non-canonical naturally occurring nucleobase, such as e.g. chemically modified bases or epigenetic bases, may be comprised within a nucleic acid sequence (tag) in the context of the present invention. By extending the number of different types of nucleobases used in the code of the sequence tags the complexity of the code and/or the number of unique libraries that can be generated can be extended or modified.

Herein when referring to a sequence of a nucleic acid sequence tag being unique within the library, a "complete" uniqueness of the sequence of the nucleic acid sequence tag may be meant or that in embodiments the majority of the plurality of tag sequences are unique within the library, but some or few sequences may be duplicate. Such duplicate sequence "artefacts" may arise from technical errors during the *in-silico* sequence calculation and/or the nucleic acid sequence tag synthesis. Accordingly, in some embodiments a library comprising only unique nucleic acid sequence tags may still comprise some nucleic acid sequence tag "artefacts" with duplicate sequence.

This nucleic acid sequence may be biochemically and bioinformatically identified and characterized using DNA or RNA sequencing. The sequencing analysis may also involve the comparison of the obtained nucleic acid sequence and one or more reference nucleic acid sequences.

Circular nucleic acids (CNAs) are nucleic acid molecules with a closed-loop structure. This feature comes with a number of advantages including complete resistance to exonuclease degradation, much better thermodynamic stability, and the capability of being replicated in some embodiments by a DNA polymerase in a rolling circle manner. A "circular nucleic acid molecule" can refer herein to a circular DNA molecule, a circular RNA molecule, a plasmid, a vector, a DNA vector, a cosmid, circular chromosomes, circular mitochondrial DNA, circular genomic RNA or circular genomic DNA.

The term nucleotide can be abbreviated with "nt". The term base pair (two nucleobases bound to each other by hydrogen bonds) can be abbreviated with "bp".

Herein a "nucleic acid sequence tag" is a short nucleic acid sequence or a short nucleic acid sequence fragment that is preferably used to mark, tag, characterize and/or identify a substrate. The terms "nucleic acid sequence tag", "nucleic acid fragment", "fragment", "barcode" or "tag" may be used herein interchangeably.

Herein, the exact consecutive nucleotide sequence of a nucleic acid may also be referred to as the "sequence identity" of said nucleic acid. A shared sequence identity means herein that, for example, two nucleic acids share the exact same sequence of a certain number of consecutive nucleotides, which may also be referred to as 100 % sequence identity. Lower percentages of sequence identity relate to a non-perfect sequence identity of the nucleic acid sequence, wherein the indicated percentage of the sequence is identical.

The herein recited "equal distribution" or "nearly equal distribution" of "nucleic acid bases in each sequence position of the entirety of nucleic acid sequences comprised within the library" can be described in other words as follows. First, all nucleic acid sequence tag molecules comprised within the library can be referred to as the "entirety" of nucleic acid sequence tag molecules comprised within the library. The following description is meant to describe a specific embodiment of the invention to illustrate how the invention can be carried out and is not intended to limit the scope of the invention. Said "all nucleic acid sequence tag molecules", no matter if they are comprised within a circular nucleic acid or not, have the same length, e.g. in some embodiments 100 consecutive nucleotides. Accordingly, their nucleic acid sequence has in said embodiment a first to a hundredth sequence position wherein each position comprises a certain nucleobase e.g. A, T/U, C or G or others. In the "random sequences" of the tags (the sequence outside/surrounding the characteristic positions) the distribution of nucleotide bases is already, due to their random computer-based selection, nearly equal or equal within each sequence position, as any base has the statistically same chance (plus/minus the statistical error) of being chosen in a random sequence. Hence, only the bases in the characteristic positions are excluded from this principle, as they are pre-defined and not randomly chosen. If the distribution of the nucleic acid bases in the characteristic position would not be balanced within the library in embodiments of the invention either by a) the ratio in which different groups of tag sequences (with different bases in the characteristic positions) are added to the library or b) by additional groups of tag sequences, a forger might identify the location and hence pattern of the characteristic positions by simply sequencing the tag library embedded in the substrate and by looking for positions that don't have an equal distribution of nucleic acid bases. Accordingly, in embodiments the equal distribution of nucleic acid bases in the characteristic positions of the entirety of tag sequences comprised within the library is ensured by the specific combination of different groups of tags according to their nucleic acid bases in the characteristic positions. In a specific example of an embodiment, if a person aiming to validate the substrate, e.g. the customer or the valid producer/distributor on behalf of the customer, knows that the library comprises, in embodiments for example: five different groups of tags (see Fig. 2), wherein each tag comprises three characteristic positions, wherein the different groups of tags are comprised within the library in a specific ratio to each other. Further, in said embodiment the validating person knows that the characteristic positions are located e.g. in sequence position 20, 50 and 63, wherein tag group 2 possesses in the first characteristic position an A and in the second characteristic position a T and in the third characteristic position an A (ATA), tag group 3 would have ACT, group 4 AGC and tag group 5 AAG (e.g. see example in Fig. 2) in their characteristic positions. Form this pattern also the combinations possible in the tag group 1 (labelled with the number 1 in Fig. 2) that was added to balance the nucleic acid base ratio of the characteristic positions is known. With this knowledge the characteristic positions or the tag sequences can be analyzed by PCR, sequencing or other techniques and successfully validated. Accordingly, the sequences analyzed from the library should always have the pre-defined combinations of nucleic acids in the characteristic positions (in this example embodiment sequence position 20, 50 and 63) within each tag group, e.g. ATA or ACT or AGC or AAG and for example never AGG or ACC. The forger, on the other hand, does not know the location, number, ratio and code of the characteristic positions in a library and can also not identify them simply by sequencing, as the characteristic positions are masked within the random sequence in which they are embedded and at the same time by the equal distribution of all nucleotides in all sequence positions within the entire library. Hence, the encoded pattern can only be decoded, if the specific pattern of the characteristic positions (the code "key" so to say) is known and the person knowns what he is looking for.

In the context of nucleic acid sequencing the term "base calling" usually refers to the conversion of sequencing raw data to nucleic acid sequences.

"Polymerase chain reaction" (PCR) is a method widely used to rapidly make millions to billions of copies (complete copies or partial copies) of a specific DNA sample, allowing the amplification of a very small sample of DNA to a large enough amount. PCR amplifies a specific region of a DNA strand (the DNA target sequence), depending on where the used primers bind to start the amplification reaction. Almost all PCR applications employ a heat-stable DNA polymerase enzyme, such as Taq polymerase. Quantitative PCR (qPCR) or real-time PCR a specific form of PCR is a standard method for detecting and quantifying a specific target sequence, or quantifying gene expression levels in a sample. In qPCR, fluorescently labeled probes or nucleic acids, or fluorescent double-stranded DNA-binding dyes are incorporated into the PCR reaction and product formation is monitored in real time following each PCR cycle.

Sequencing herein can be accomplished with any sequencing technique known in the art, e.g. for RNA and/or DNA sequencing. A non-limiting list of nucleic acid sequencing techniques that can be used, alone or in combination with each other, in the context of the present method comprises methods selected from the group comprising Sequencing by Synthesis, Pyrosequencing, Sanger-Sequencing, Maxam-Gilbert sequencing, Whole-genome shotgun sequencing, Targeted-Sequencing, Clone by clone sequencing, Next-generation sequencing, Nanopore sequencing, Long-read sequencing methods, Single molecule real time (SMRT) sequencing, Short-read sequencing methods, Massively parallel signature sequencing (MPSS), Polony sequencing, 454-pyrosequencing, Illumina (Solexa) sequencing, Combinatorial probe anchor synthesis (cPAS), SOLiD sequencing, Ion Torrent semiconductor sequencing, DNA nanoball sequencing, Heliscope single molecule sequencing, and Microfluidic Systems Sequencing.

CRISPR gene editing is a genetic engineering technique in molecular biology by which the genomes of living organisms may be modified. It is based on a simplified version of the bacterial CRISPR-Cas9 antiviral defense system. By delivering the Cas9 nuclease complexed with a synthetic guide RNA (gRNA) into a cell, the cell's genome can be cut at a desired location, allowing existing genes to be removed and/or new ones added in vivo (in living organisms). This technique can also be applied for (CRISPR)/CRISPR-associated systems (Cas)-assisted nucleic acid detection assays. The advantages of the (CRISPR)/CRISPR-associated systems (Cas), e.g. DETECTR, SHERLOCK and STOP or other digital CRISPR/Cas-Assisted assays (Pak et al., Adv. Sci. 2021; Kellner at al., Nat Protoc, 2019; Mustafa et al., J. Clin. Microbiol., 2021), are their fast turnaround time, and potential circumvention of instrument-intensive thermocycling. In some of such assays the detection of a target sequence, e.g. in the context of the present invention a motif of a characteristic position, can be achieved by Cas-mediated (e.g. Cas12) detection of predefined target sequences, after which cleavage of a reporter molecule confirms detection of the virus.

Herein a "sample" may be taken from any marked item, product or substrate. Preferably said sample comprises nucleic acid molecules, even more preferably nucleic acid sequence tag molecules that were applied to/are present on said substrate.

Herein "marking" or "tagging" may refer to the application of a nucleic acid sequence tag according to the invention to a substrate or product, referring to the labelling or tagging of said substrate with the tags or tag library according to the invention. A mark, tag or label according to the invention can at a later timepoint be extracted, sampled or obtained entirely or partially from the before marked substrate. Preferably neither the marking (application of the tag (library)) nor the sampling causes any damage or loss in value of the substrate.

Herein a "library" or "tag library" refers to a plurality (pool) of nucleic acid sequence tags according to the invention. Said library may in certain embodiments comprise two or more different groups of nucleic acid sequences tags, wherein each group of nucleic acid sequence tags itself comprises at least two, preferably more than two nucleic acid sequence tag molecules and wherein the nucleic acid sequences tags within one group comprise the same combination of nucleic acid sequence motifs or "code" encoded within their characteristic positions. The nucleic acid sequence tags within one group of tags preferably comprise the same number of characteristic positions, preferably at least two characteristic positions. In other words, the motifs within the characteristic positions and the combination and location of said characteristic positions within the nucleic acid sequence tag are characteristic for one group of nucleic acid sequence tag. In embodiments all nucleic acid sequence tags within a library comprise the same number of characteristic positions. In one preferred embodiment a library comprises only or majorly tags with unique nucleic acid sequences. Said uniqueness of the sequences within the library might in embodiments be due to the majority of each tag nucleic acid sequence being chosen randomly (besides characteristic positions). In some embodiments, the sequences within a library might be chosen or selected in advance *in silico* to be unique within the library.

Herein a "characteristic position" is the sequence position of a motif within the nucleic acid sequence of a tag molecule. The position (sequence position given in bp or nt) and motif of a characteristic position herein is known to the producer, provider or vendor of a marked substrate/product and is used to encode a characteristic pattern or code to identify said substrate/product. To achieve a sufficiently high complexity and security of the code at least two, a first and a second, characteristic position should be comprised within a nucleic acid sequence tag according to the invention. In some embodiments a sequence tag comprises more than two, for example a third and a fourth characteristic position, which results in an increased complexity and security of the code/pattern. In some preferred embodiments the first characteristic position is in the same sequence position in each nucleic acid sequence tag of one or more or of all groups of nucleic acid sequence tags within a library. In some embodiments the same applies for the second and the optionally further characteristic positions. As a non-limiting example of one embodiment, the first characteristic position is in each tag within the library, for example, in sequence position 10, and the second characteristic position is, e.g., in the sequence position 20 of each nucleic acid sequence tag comprised within the library. For example, in embodiments, the first characteristic position within one or more groups of nucleic acid sequence tags within a library are at a defined sequence position within the nucleic acid sequence tag, wherein the second characteristic position within one or more groups of nucleic acid sequence tags within a library is at another defined sequence position within the nucleic acid sequence tag. Hence, in said embodiments the position of the characteristic positions within a library of nucleic acid sequence tags is known to the designer of the code, the provider or distributor and can be used to encrypt and decode the library code for validation.

Herein a "motif" refers to an array of nucleic acids, wherein herein a motif comprises an array of at least one nucleic acid, in some embodiments a motif comprises arrays of at least two consecutive nucleic acids. Accordingly, in some embodiments a motif may refer to a single nucleic acid, in other embodiments a motif may refer to more than one consecutive nucleic acids. The combination of specific motifs in the at least first and the at least second characteristic positions of a nucleic acid sequence tag can be used to encode a characteristic code, that can be used for identification of a substrate to which the library of tags is applied. Examples of modes of execution of embodiments of the present invention can be found in the examples and the Figures.

In the context of the present method, the terms "design", "pattern" or "code" may be used interchangeably when referring to the pattern encoded by the specific motifs and the characterizing positions of nucleic acid sequence tags.

Unless defined otherwise, all technical and scientific terms used herein have the same meaning as commonly understood by one of ordinary skill in the art to which this invention belongs. The practice of the present invention will employ, unless otherwise indicated, conventional techniques of chemistry, molecular biology, microbiology, recombinant DNA technology, and bioinformatics. Bioinformatics and sequencing terms, unless otherwise indicated herein, have the meaning as described, for example in Baxevanis et al. 2004, Bioinformatics.

## FIGURES

The invention is further described by the following figures. These are not intended to limit the scope of the invention but represent preferred embodiments of aspects of the invention provided for greater illustration of the invention described herein.

### Brief description of the figures:

**Figure 1:** Embodiment where the nucleic acid sequence tags comprise single nucleotides in the characteristic positions and the library is embedded into a painting, artwork or collectible. In this embodiment the tags are incorporated within circular nucleic acids, such as plasmids.

**Figure 2:** Embodiment where the nucleic acid sequence tags comprise single nucleotides in the characteristic positions and the library comprises five different groups on nucleic acid sequence tags.

**Figure 3:** Embodiment where the nucleic acid sequence tags comprise a motif of more than one, here an array of eight, nucleotides in the characteristic positions and the library is embedded into a substrate in or on the packaging of a product.

**Figure 4:** Example of the computation and selection of the nucleic acid combinations (patterns) in the characteristic positions of nucleic acid sequence tags in two specific embodiments.

**Figure 5:** Example design of tags with a length of 10 bp for increasing numbers of characteristic positions.

**Figure 6:** Example design of tags with a length of 20 bp for increasing numbers of characteristic positions.

**Detailed description of the figures:**

**Figure 1:** The figure depicts one non-limiting embodiment of the marking of an item, such as a painting or testament according to embodiments of the method of the present invention. The figure depicts an example of embodiments where the nucleic acid sequence tags comprise single nucleotides in the characteristic positions, wherein nucleic acid molecules are embedded in the paper/material of e.g. a painting, artwork or collectible. Each library (in the scheme the two different libraries are depicted by two different tag molecules with the upper strand representing the first library and the bottom strand representing the second library) includes one of two patterns with two characteristic positions (arrow marker) among 100 bases of randomized nucleic acid (here DNA) sequence. Each library of nucleic acid sequence tag molecules consists of many nucleic acid sequence tag molecules different from each other due to the randomness of each position (each N can be A, T, C or G in an equal distribution). Only in two selected characteristic positions the distribution of nucleotides is intentional. In the first library, depicted as the top/upper strand in the scheme, 98 bp are randomly chosen between numerous samples while the characteristic position 1 only contains A or T and characteristic position 2 only comprises C or G. The second library, which is depicted on the bottom of the scheme, will have the opposite design (position 1: C or G, position 2: A or T). Applying both libraries with the same proportion to the substrate will result in an equal distribution of nucleic acids in all positions. In the depicted specific example each 100 bp nucleic acid sequence tag molecule is embedded inside a plasmid (a circular nucleic acid molecule) before being added to the paper/material. To verify/validate and approve an item, the embedded plasmids should be isolated and sequenced. All analyzed plasmids should follow the design. If this is not true, the product is forged.

**Figure 2:** The figure depicts an embodiment wherein a library comprises four different groups of nucleic acid sequence tags (depicted as tag molecules/strands with the numbers 2-5), which comprise single nucleotides in their characteristic positions. The figure depicts an embodiment wherein the single nucleotides in the characteristic positions encode a three-position pattern. In this example, four groups of tags (shown in the scheme as strands 2-5) follow a specific design. As an example, if the first characteristic position has the nucleic acid base A and the second position has T, the third position needs to show A (tag group 2). The other tag groups (numbered in scheme as strands 3 to 5) also follow the depicted design. To balance the about equal distribution of the nucleic acid bases in each sequence position of the entirety of nucleic acid sequences comprised within the library, especially in the characteristic positions, a fifth group of nucleic acid sequence tag molecules (tag/strand on the top, numbered as strand 1) is added to the library in a specific ratio to the other groups (12/16 vs. 4x(1/16)). This group of tag sequences (numbered 1 in the scheme) should comprise respective nucleic acid bases in the three characteristic positions (arrow-shaped markers on top (1, 2, 3)) to balance the ratio of nucleic acid bases comprised within the characteristic positions of the other four tag groups (strands 2-5 in the scheme). Accordingly, the fifth group (number 1 in the scheme) of nucleic acid sequence tags then should be added to the substrate 12 times more than each other group (strands 2-5 in scheme) to get an equal distribution of A, T, C and G in each sequence position if the whole library is analyzed. To validate a product, one needs to sequence the nucleic acid molecules embedded in the product (substrate) in order to check if the embedded nucleic acid library follow this design. Due to the about equal distribution of nucleic acid bases in each sequence position of the entirety of nucleic acid sequences comprised within the library it is difficult for a forger to determine the location and number of the characteristic positions and hence to imitate the characteristic pattern encoded therein.

**Figure 3:** An example of embodiments of the invention using arrays of several nucleotides (here 8) in the characteristic positions, wherein the coded pattern encoded in the nucleic acid library is used along with an additional feature for online verification. A: In this design, there is a label embedded on the packaging of the product with three spots and each spot contains a distinctive library of nucleic acid molecules. In this example, the first and last spot of the label include nucleic acid sequence tag molecules comprising nucleic acid sequence tags from a first group having in the characteristic position the sequence depicted in Fig. 3B (1). The middle spot contains nucleic acid sequence tag molecules of a second group having in the characteristic position the sequence depicted in Fig. 3B (2). For validation a CRISPR-Cas system can be used, which is "programmed" to give a positive result for group 1, revealing the pattern "positive-negative-positive". This pattern then could be checked online. If the pattern used for this product marches the online verification system, the originality of the product would be validated. B: In this specific embodiment the coded pattern of the characteristic position is based on a specific combination of different arrays of multiple DNA nucleotides (4 arrays of each 8 nucleotides) instead of single nucleotides. If the coded pattern includes the correct combination of these arrays (1 and 2 in the example shown in 3B) a "programmed" sgRNA from a Cas9-based validation kit, which is provided for validation of the product, can bind to the tag sequence and gives a positive result for those tags/library including the array combination 1-2 (tag sequence 1 in Figure 3B). If a fragment does not contain the combination of the arrays 1-2 (tag sequence 2 in Figure 3B), the sgRNA cannot bind resulting in a negative output signal.

**Figure 4:** A non-limiting example of the computation and selection of the nucleic acid combinations (patterns) encoded by the characteristic positions of the nucleic acid sequence tags according to embodiments of the invention. If in said embodiments a pattern of two characteristic positions (top of Fig 4) is chosen, 16 unique combinations of nucleic acids in the characteristic positions are possible. To increase the degree of encryption in embodiments only specific combinations of nucleic acids in the characteristic positions can be chosen. In the present example, the nucleic acid sequence tag comprises 20 nucleotides, from which 18 are chosen randomly and 2 are characteristic positions, which comprise the nucleic acid combinations of CA, TC, GA, AC, AG, GT, TG and CT (8 groups of different tags). The nucleic acid combinations excluded from the code in the characteristic positions are depicted on the upper right of Figure 4. In this embodiment, the number of nucleic acid sequence tag molecules that need to be analyzed to achieve certainty of validation of 99.9% would be 10 (A). The certainty of validation in embodiments can be calculated according to the following formula (1- (R^A))*100, wherein the number of characteristic positions is 2, the possible combinations: 4 $bases^2$ (4*4)= 16, the valid combinations are chosen to be 8, therefore the ratio R of valid to possible combinations is R= 8/16 = 0.5. So, A=10 nucleic acid sequence tag molecules need to be analyzed for a validation certainty of 99.9%. While A shows the number that needs to be analyzed to verify that a library of DNA sequences matches the known pattern, F gives the minimum number of nucleic acid sequence tag that need to be analyzed to decode the pattern if the pattern is not known (e.g. by a forger). F is based on the maximum number of sequences whereby below this number, the characteristic positions cannot be found to be unique. So, by analysing the relationships between the respective two or three characteristic positions, there can be found more than one combination that is missing the number of nucleotide combinations equal or more than the characteristic positions missing combinations. The same calculation is depicted on the bottom half of Figure 4 for an embodiment in which a tag library comprises three characteristic positions, wherein the 12 nucleic acid combinations that are excluded from the code that is encoded in the characteristic positions are depicted on the bottom right of the figure.

**Figure 5:** The Figure depicts an example of a design where half of the possible combinations in the characteristic positions are missing. This example includes: the calculated upper threshold (the number of nucleic acid tag sequences / fragments where above that the forger might be able to decipher the code), the lower threshold (the number of nucleic acid tag sequences / fragments required to validate the code), and the space in between that can be used for embodiments of the tagging method according to the invention. In the graph, for fragments of 10 bp, the number of fragments (Y) required to decipher the code for a forger is shown for sequence tags with an increasing number of positions (X). As shown in the graph, there is a clear exponential trend line, and using such a trend line it is possible to predict the number of required fragments for embodiments with a higher number of characteristic positions that could not be calculated. Using the trend line from 10 bp long nucleic acid sequence tags, it is possible to calculate the proximity of the required fragments for a higher number of positions. So, with 25 or 26 characteristic positions, more than 10^14 fragments would be required, which are close to the number of fragments in a 1ng of DNA sample. Considering that it would still be possible to analyse only 10 fragments to validate the design, this number of unique fragments would help with increasing the security and difficulty of copying and cracking the code encrypted within the tags. Increasing the number of positions to 30 would result in 1000 ng of unique fragments. So, if 1 ng of fragments are added to each product, at least 1000 products could use a specific design with 30 positions, and none of them would share an identical sequence of DNA.

**Figure 6:** The figure depicts an example of a design where half of the possible combinations in selected positions are missing. In the graph, for fragments of 20 bp, the number of fragments (Y) required to find the design for a forger is shown for designs with an increasing number of positions (X). This example includes: the calculated upper threshold (the number of fragments where above that the forger might be able to decipher the code), the lower threshold (the number of fragments required to validate the code), and the space in between that can be used for embodiments of the tagging method according to the invention. In the graph, for fragments of 20 bps, the number of fragments (Y) required to decipher the code by a forger is shown for sequence tags with an increasing number of positions (X). As shown in the graph, there is a clear exponential trend line, and using such a trend line it is possible to predict the number of required fragments for embodiments with a higher number of characteristic positions that could not be calculated. Using the trend line from 20 bp long nucleic acid sequence tags, it is possible to calculate the proximity of the required fragments for a higher number of positions. So, with 25 or 26 characteristic positions, more than 10^14 fragments would be required, which are close to the number of fragments in a 1 ng of DNA sample. Considering that it would still be possible to analyse only 10 fragments to validate the design, this number of unique fragments would help with increasing the security and difficulty of copying and cracking the code encrypted within the tags. Increasing the number of positions to 30 would result in 1000 ng of unique fragments. So, if 1ng of fragments are added to each product, at least 1000 products could use a specific design with 30 positions, and none of them would share an identical sequence of DNA.

## EXAMPLES

The invention is further described by the following examples. These are not intended to limit the scope of the invention but represent preferred embodiments of aspects of the invention provided for greater illustration of the invention described herein.

### EXAMPLE 1

The Figure 4 shows examples of two embodiments with a different number of characteristic positions comprising a single-nucleotide-motif, one example comprises two (top) the other three (bottom of Figure 4) characteristic positions. The Figure also shows the ratio (R) of the nucleotide combinations in the characteristic positions that were not used/excluded from the design to the total number of combinations possible with respective motifs in the characteristic positions.

In the first example (with properties mentioned in the box on the top left of Figure 4), a tag design comprising two characteristic positions is used wherein each fragment of these two characteristic positions should have a known combination of nucleotides. In 18 other positions, nucleotides are randomly chosen and are not used for the validation or encryption of the pattern. So, each sequence of nucleotides constructed based on this design (e.g. while excluding the 8 nucleic acid combinations, enlisted on the top right side of Figure 4) will have one of 8 possible combinations in the characteristic positions. On the other hand, in a completely random generated pattern, a total of 16(4*4) combinations should be present in the characteristic positions.

In the second design (box on the bottom left of Figure 4), three characteristic positions are chosen for the tag design, with 12 (enlisted on the bottom right side of Figure 4) out of 64 possible nucleic acid combinations in these characteristic positions are missing/excluded from the designed pattern/code and 52 nucleic acid combinations are present/used for the code.

The validation procedure is based on the number of nucleic acid sequence tag molecules (A) that needs to be analyzed to assure that the substrate is marked with the correct library (the substrate is the valid product). While A shows the number, that needs to be analyzed to verify that a library of nucleic acid sequences matches the known pattern, F gives the minimum number of fragments required to find the pattern if the pattern is not known. F is based on the maximum number of sequences whereby below this number, the designed positions cannot be found to be unique. So, by analyzing all the respective two- or three-position relationships, there can be found more than one combination that is missing the number of nucleotide combinations equal or more than the characteristic positions missing combinations.

In the case of the pattern of two characteristic positions. Each correct sequence, with a 50% of chance, is from a correct library (correct substrate/product) and 50% from a random library (forged product). With 10 validated tag sequences (and no sequence comprising the eight in Figure 4 (top right) enlisted nucleic acid combinations), there is less than a 0.1% chance that these 10 sequences are all form a completely random library (forged product).

<u>Formula</u>: certainty for the correct validation of a unique sequence library = (1- (R^A))*100

<u>Embodiment with 2 characteristic positions:</u>

Positions: 2

Possible combinations: 4 bases$^2$ (4*4): 16
Valid combinations: 8
Ratio (R): 8/16 = 0.5
F:18
A:10

<u>Calculation</u>:

$$0,5^{10} = 0.00097$$

1-0.00097= 0.99903 = 99.9%

<u>Embodiment with 3 characteristic positions:</u>

Positions: 3

Possible combinations: 4 bases$^3$ (4*4*4): 64
Valid combinations: 52
Ratio (R): 52/64= 0.8125
A=34
F=170

<u>Calculation</u>:

$$0.8125^{34}= 0.00086$$

1-0.00086= 0.99914 = 99.9%

This number for the three-position design is 34:
52/64 = 0.81725

Each correct sequence can be 81.725% from a random library. So:

$$0.81725^{34} = 0,00086$$

1-0,00086= 0.99914 = 99.9%

With 34 correct tag sequences, one can be sure that with 99,9% accuracy the analyzed tag sequences are from the valid designed library.

This calculation is used to generate a given number of sequences with the desired tag design. By analyzing the generated sequences, different position-related combinations are recorded, and they are compared to the characteristic positions. The maximum number of sequences is found whereby generating more than this number, the characteristic position will be the position that is missing the maximum number of combinations.

As an example, for a tag design with three characteristic positions with less than 170 different sequences, there is more than one three-position that is missing 12 or more than 12 combinations of three nucleotides. As it is shown in Fig. 4, by changing the number of positions and excluded/total ratio, the upper (F) and lower (A) threshold and consequently the space between two thresholds will change. This allows to find the optimum number of required sequences in each application by using an alternative number of positions and combinations of nucleotides and the length of the sequence.

**EXAMPLE 2**

One execution example of embodiments of the present invention is the marking of paintings to prevent them from plagiarism. In this case, the library of nucleic acid molecules needs to be added to the drawing paper, canvas or ink/paint in a way that ensures an easy and nondestructive sample collection any time in the future. This means that the coded nucleic acid, e.g. DNA, applied at any time to a product needs to have a stable encoding capacity even in a hundred years, and beyond, independent of possible technical progress. At a certain time in the future, if someone wants to prove the authenticity of the painting (or another product), nucleic acid, e.g. DNA, molecules will be isolated from the paper (or ink), multiplied (if necessary), and analyzed via sequencing or other upcoming future technologies for nucleic acid analysis. When knowing the code, one can easily identify if the DNA library under investigation is the original library, or a forgery. For someone not knowing the code, it is not possible to crack/identify the code as more molecules are necessary to identify the code (do the computational analysis) than present on the product.

Other exemplary execution examples for these embodiments would be any high-value product such as paintings, art prints, other artwork, certificates or testaments. For non-printed products, such as jewelry, artwork or collectibles, the library may be applied with a suitable carrier or sealant, such as e.g. a glue, plastic or resin.

## EXAMPLE 3

Another execution example of embodiments of the present invention would be the protection of pharmacological products, expensive biotechnological substances or kits. In this example, a fast and low-price identification of the authenticity of the nucleic acid library needs to be possible in high throughput on a regular basis. Furthermore, the nucleic acid code needs to be safe for only a restricted time covering the life span of such a product (e.g. 1-2 years). For this reason, the included pattern is less complex in comparison to the tags described in the previous examples. The authenticity of the product might be proven by a simple dCas9-based test kit incorporating sgRNAs specifically binding to the correct motif (a defined nucleic acid sequence pattern). If the target sequence is available, the sgRNA binds the sequence and this will result in an enzymatically catalyzed color shift as an easy read-out. For this kind of detection, already published detection methods can be used (Chen et al., Science, 2018, Kellner et al., Nature protocols, 2019). Such test kits could be applied in a rapid and cost-effective manner without specific knowledge. The execution of the testing is comparably simple, similar to known rapid test kits of the prior art.

Execution examples for these embodiments would be any consumables worth protecting like pharmaceuticals, expensive laboratory consumables, wine bottles, cigarettes or cigars, luxurious fashion items.

## EXAMPLE 4

The present example shows an embodiment of the invention implementing the features of the method according to the invention to achieve a superior protection from counterfeiting. The present example illustrates how in embodiments the upper threshold (the maximum number of tag molecules that should be applied to a substrate, such that the applied library is below a threshold of tag molecules required by a forger to identify the unique code encoded in the library) and the lower threshold (the number of tag molecules required to validate the library), can be calculated and applied for the provision of a secure tagging library according to the invention.

In the present example the motifs in the characteristic positions comprise a single nucleotide. From all possible nucleotide combinations between the characteristic positions, herein only half of the possible combinations are used within the library. In the graphs in Figure 5 and 6 (showing tag molecules with either 10 or 20 bps) the number of nucleic acid sequence tag molecules (Y) required to decipher the code of the library by a forger is shown for nucleic acid sequence tags with an increasing number of characteristic positions (X). As shown in the graphs, an exponential trend line is obtained, which can be used to predict the number of the maximum number of nucleic acid sequence tag molecules that should be applied to a substrate.

Using the trend line from 10 bp (Fig. 5) and 20 bp (Fig. 6) nucleic acid sequence tags, the number of the tag molecules required for deciphering the code in case of a higher number of positions can be estimated. As the present embodiment calculates *in silico* the sequences comprised within a library the exact number of molecules to be analyzed for decryption can be estimated.

Accordingly, with 25 or 26 characteristic positions more than 10^14 unique tag molecules would be required which are close to the number of tag molecules making up 1ng. Considering that 10 tag molecules are necessary for successful validation of the library, this number of unique tag molecules would increase the security and difficulty of copying and/or identifying the library code by a forger. Increasing the number of characteristic positions to 30 would result in 1000 ng of unique tag molecules. So, 1ng of tag library is added to a product, at least 1000 products be marked uniquely with a specific tag sequence design comprising 30 characteristic positions, and none of them would share an identical nucleic acid sequence. This practical example shows an specific embodiment of a tag design that not only encodes a secure code but also utilizes a security check analysing the proportion of unique sequences" with relatively cheap and fast synthesized oligonucleotide tag sequences.

The present example also illustrates the improvement provided by the invention in view of the prior art. US2021/0108194A1, for example, uses in one approach randomly selected libraries accompanied by overhang sequences used for primer binding. This can limit the variety of libraries and make copying easier as one can use the unique primer sequences and replicate sequences associated with them. In another approach, US2021/0108194A1 uses a known sequence ("barcode"). This is also reducing the security provided against fortifying by the same way as primer binding sites. The number of different barcodes is relatively limited in the prior art approach and is the same among several species, so it will be easy for a forger to identify them by sequencing. Finding a barcode can result in finding the "Random identifier library" or RIL (library of species (unique sequences) associated with a tag) related to it.

In the present approach, although nucleic acid sequence tags are used, it is nearly impossible to identify the code encoded in a tag sequence according to the invention by PCR, as most of the tag sequence is random and unique for a fragment and the characteristic positions are too short for primer attachment. And due to the additional encoding or protection of the code/pattern encoded in the characteristic positions by the surrounding random sequence the encoded pattern is still secure even if one analyses all sequences of a library that were applied to a substrate. This combination of features and the surprising effect of achieved superior security of the tagging approach according to the invention are neither disclosed nor suggested by any one of the prior art methods. Furthermore, as the encryption in the present approach is done on the nucleotide synthesis level, PCR or primer attachment sides are not required for tag or library construction.

Another shortcoming of US2021/0108194A1 is that each nucleotide of the barcode sequence represents specific information. So, every nucleic acid sequence in a barcode is important for retrieving the information. However, in the present approach, it is not necessary for secure encryption to encode any information into the randomly chosen part of the sequence. The present approach only requires the nucleic acid motifs and their combinations in the characteristic positions.

Further, in US2021/0108194A1 for the creation of an identifier from random sequences, there is a need to select a minimum number of random sequences and assign them to an identifier. This number is limited to the technological limitations and the error threshold of the isolation step. So, the variety of unique fragments are a small fraction of the maximum potential number of molecules made out of randomized sequences. In embodiments of the present approach, each nucleic acid sequence tag molecule comprises a unique randomized sequence and at the same time the pattern encoded by the characteristic positions. Although the verification and decoding of the library according to the invention needs a minimum and a maximum number of sequences that should be comprised, the combinations of nucleobases in the characteristic positions that are not used for the tags/within the library can be as small as one. So, theoretically, the variety of tag molecule sequences can be very close to the possible number of different randomized sequences. This will drastically increase the flexibility and utility of the design of the tag sequences as the present approach facilitates the use of a highly randomized library that makes the identification and replication of the encoded pattern or code considerably difficult but has no effect on the validation process.

## Claims

1. Method of marking a substrate with a unique library of nucleic acid sequence tags comprising:

    a. providing a library of nucleic acid sequence tags, comprising at least two different groups of nucleic acid sequence tags that each comprise a plurality of nucleic acid sequence tags,

        wherein each of said nucleic acid sequence tags comprises a defined nucleic acid motif in at least a first and a second characteristic position, and wherein outside of said at least two characteristic positions each nucleic acid sequence tag comprises a random nucleic acid sequence,
        wherein the combination of the nucleic acid motifs of the at least first characteristic position and the at least second characteristic position of a nucleic acid sequence tag is characteristic and unique for the group of said nucleic acid sequence tag, and

    b. marking the substrate with the unique nucleic acid sequence tag library by applying the nucleic acid sequence tag library to the substrate.

2. Method according to claim 1, wherein the entirety of nucleic acid sequence tags comprised within the library comprises in each nucleic acid sequence position each type of nucleic acid base which is comprised within the library at an equal ratio +/- a margin of error relative to the number of total fragments in the entire library and the length of the random sequence of each nucleic acid sequence tag.

3. Method according to any one of the preceding claims, wherein only specific combinations of nucleic acid motifs in the at least first and the at least second characteristic position are used within the nucleic acid sequence tags.

4. Method according to any one of the preceding claims, wherein,
if each characteristic position comprises a motif that is only one nucleotide in length, each type of nucleic acid base, which is comprised within the library, is present at an equal ratio within each of the at least first and the at least second characteristic position of the entirety of nucleic acid sequence tags comprised within the library, as

    a. the nucleic acid bases in the at least first and the at least second characteristic position of the nucleic acid sequence tags are selected accordingly and/or the groups of nucleic acid sequence tags are comprised within the library at a specific ratio, or
    b. a sufficient amount of additional nucleic acid sequence tags which comprise selected nucleobases in the at least first and second characteristic position is comprised within the library.

**5.** Method according to any one of the claims 1 to 3 wherein the nucleic acid motif in the at least first and the at least second characteristic position each comprises a nucleotide sequence of at least two consecutive nucleotides.

**6.** Method according to any one of the preceding claims, wherein at least 80%, preferably at least 90%, of the nucleic acid sequence tag molecules within the library comprise a nucleic acid sequence that is unique within the entire library.

**7.** Method according to any one of the preceding claims, wherein the amount of library that is applied to the substrate comprises 10 to 10^15 times, preferably 100 to 10^14 times, the number of nucleic acid sequence tag molecules that have to be analyzed (A) from the applied library for a specific certainty of correct validation (V), wherein the specific certainty of correct validation (V) is calculated according to the formula:

$$V = (1- (R^A))*100,$$

wherein R is the ratio of the number of unique combinations of nucleic acid motifs at the at least first and second characteristic positions comprised within the library over the number of possible unique combinations of motifs.

**8.** Method according to any one of the preceding claims, wherein each nucleic acid sequence tag is present as a circular nucleic acid molecule or is comprised inside a circular nucleic acid molecule.

**9.** Method according to any one of the preceding claims, wherein a substrate is selected from the group of paper, cardboard, canvas, ceramic, cotton, wool, leather, nylon, plastic, vinyl, stone, metal, glass, liquid, a pharmaceutical, a packaging article, sculptures, paintings, artwork, computers, computer peripheral devices, printers, microchips, televisions, sound systems, furniture, appliances, antiques, clothing, handbags, shoes, sunglasses, cameras, 3-D printed objects, automobiles, bicycles, motorcycles, luggage or collectibles.

**10.** Method according to any one of the preceding claims, wherein the library of nucleic acid sequence tags that is applied to the substrate is configured to be analyzed

a. by extracting nucleic acid sequence tags of the library from the substrate and analyzing the nucleic acid sequence tags, or
b. by analyzing nucleic acid sequence tags of the library directly on the substrate.

**11.** Method according to claim 10, wherein analyzing the nucleic acid sequence tags comprises performing one or more of the analysis methods selected from the group comprising nucleic acid sequencing, nucleic acid restriction assays, hybridization assays, immunoassays, CRISPR-Cas assays, molecular beacon assays, PCR, digital PCR, qPCR and real-time PCR.

**12.** Packaging article, wherein the packaging article comprises a substrate that has been marked according to the method of claims 1-11.

**13.** A kit for analyzing a library of nucleic acid sequence tags that has been applied to a substrate according to the method of claims 1-11, comprising

- means for performing one or more of the analysis steps according to the method of claim 10 and at least one of the methods according to claim 11,
- optionally means for nucleic acid extraction.

**14.** Nucleic acid sequence tag library according to claim 1 step a. for use in the method of claims 1-11.

## Patentansprüche

**1.** Verfahren zum Markieren eines Substrats mit einer einzigartigen Bibliothek von Nukleinsäure-Sequenzmarkern, umfassend:

a. Bereitstellen einer Bibliothek von Nukleinsäure-Sequenzmarkern, umfassend mindestens zwei verschiedene Gruppen von Nukleinsäure-Sequenzmarkern, die jeweils eine Vielzahl von Nukleinsäure-Sequenzmarkern umfassen,

wobei jeder der Nukleinsäure-Sequenzmarker ein definiertes Nukleinsäure-Motiv in mindestens einer ersten und einer zweiten charakteristischen Position umfasst, und wobei außerhalb der mindestens zwei charakteristischen Positionen jeder Nukleinsäure-Sequenzmarker eine zufällige Nukleinsäuresequenz umfasst,
wobei die Kombination der Nukleinsäure-Motive der mindestens ersten charakteristischen Position und der mindestens zweiten charakteristischen Position eines Nukleinsäure-Sequenzmarkers charakteristisch und einzigartig für die Gruppe des Nukleinsäure-Sequenzmarkers ist, und

b. Markieren des Substrats mit der einzigartigen Nukleinsäure-Sequenzmarker-Bibliothek durch Aufbringen der Nukleinsäure-Sequenzmarker-Bibliothek auf das Substrat.

**2.** Verfahren nach Anspruch 1, wobei die Gesamtheit der in der Bibliothek enthaltenen Nukleinsäure-Sequenzmarker in jeder Nukleinsäure-Sequenzposition jeden Typ von Nukleinsäurebase, der in der Bibliothek enthalten ist, in einem gleichen Verhältnis +/- einer Fehlermarge relativ zur Anzahl der Gesamtfragmente in der gesamten Bibliothek und der Länge der Zufallssequenz jedes Nukleinsäure-Sequenzmarkers umfasst.

**3.** Verfahren nach einem der vorhergehenden Ansprüche, wobei innerhalb der Nukleinsäure-Sequenzmarker nur spezifische Kombinationen von Nukleinsäure-Motiven in der mindestens ersten und der mindestens zweiten charakteristischen Position verwendet werden.

**4.** Verfahren nach einem der vorhergehenden Ansprüche, wobei, falls jede charakteristische Position ein Motiv umfasst, das nur einen Nukleotid lang ist, jeder Typ von Nukleinsäurebase, der in der Bibliothek enthalten ist, in einem gleichen Verhältnis innerhalb jeder der mindestens ersten und der mindestens zweiten charakteristischen Position der Gesamtheit der in der Bibliothek enthaltenen Nukleinsäure-Sequenzmarker vorhanden ist, indem a. die Nukleinsäurebasen in der mindestens ersten und der mindestens zweiten charakteristischen Position der Nukleinsäure-Sequenzmarker entsprechend ausgewählt werden und/oder die Gruppen von Nukleinsäure-Sequenzmarkern in einem spezifischen Verhältnis in der Bibliothek enthalten sind, oder b. eine ausreichende Menge an zusätzlichen Nukleinsäure-Sequenzmarkern, die ausgewählte Nukleobasen in der mindestens ersten und zweiten charakteristischen Position umfassen, in der Bibliothek enthalten ist.

**5.** Verfahren nach einem der Ansprüche 1 bis 3, wobei das Nukleinsäure-Motiv in der mindestens ersten und der mindestens zweiten charakteristischen Position jeweils eine Nukleotidsequenz von mindestens zwei aufeinanderfolgenden Nukleotiden umfasst.

**6.** Verfahren nach einem der vorhergehenden Ansprüche, wobei mindestens 80 %, vorzugsweise mindestens 90 %, der Nukleinsäure-Sequenzmarker-Moleküle innerhalb der Bibliothek eine Nukleinsäuresequenz umfassen, die innerhalb der gesamten Bibliothek einzigartig ist.

**7.** Verfahren nach einem der vorhergehenden Ansprüche, wobei die Menge der Bibliothek, die auf das Substrat aufgebracht wird, das 10 bis 10^15-fache, vorzugsweise das 100 bis 10^14-fache, der Anzahl an Nukleinsäure-Sequenzmarker-Molekülen umfasst, die aus der aufgebrachten Bibliothek für eine spezifische Sicherheit einer korrekten Validierung (V) analysiert werden müssen (A), wobei die spezifische Sicherheit einer korrekten Validierung (V) berechnet wird gemäß der Formel:

$$V = (1 - (R^A))*100,$$

wobei R das Verhältnis der Anzahl der in der Bibliothek enthaltenen einzigartigen Kombinationen von Nukleinsäure-Motiven an den mindestens ersten und zweiten charakteristischen Positionen zur Anzahl der möglichen einzigartigen Motivkombinationen ist.

**8.** Verfahren nach einem der vorhergehenden Ansprüche, wobei jeder Nukleinsäure-Sequenzmarker als ein kreisförmiges Nukleinsäuremolekül vorliegt oder innerhalb eines kreisförmigen Nukleinsäuremoleküls enthalten ist.

**9.** Verfahren nach einem der vorhergehenden Ansprüche, wobei ein Substrat ausgewählt ist aus der Gruppe bestehend aus Papier, Pappe, Leinwand, Keramik, Baumwolle, Wolle, Leder, Nylon, Kunststoff, Vinyl, Stein, Metall, Glas, Flüssigkeit, einem Pharmazeutikum, einem Verpackungsartikel, Skulpturen, Gemälden, Kunstwerken, Computern, Computerperipheriegeräten, Druckern, Mikrochips, Fernsehern, Soundsystemen, Möbeln, Haushaltsgeräten, Antiquitäten, Kleidung, Handtaschen, Schuhen, Sonnenbrillen, Kameras, 3D-gedruckten Objekten, Automobilen, Fahrrädern, Motorrädern, Gepäck oder Sammlerstücken.

**10.** Verfahren nach einem der vorhergehenden Ansprüche, wobei die auf das Substrat aufgebrachte Bibliothek von Nukleinsäure-Sequenzmarkern dafür konfiguriert ist, analysiert zu werden

a. durch Extrahieren von Nukleinsäure-Sequenzmarkern der Bibliothek von dem Substrat und Analysieren der Nukleinsäure-Sequenzmarker, oder
b. durch Analysieren von Nukleinsäure-Sequenzmarkern der Bibliothek direkt auf dem Substrat.

**11.** Verfahren nach Anspruch 10, wobei das Analysieren der Nukleinsäure-Sequenzmarker das Durchführen eines oder mehrerer der Analyseverfahren umfasst, die ausgewählt sind aus der Gruppe umfassend Nukleinsäuresequenzierung, Nukleinsäure-Restriktionsassays, Hybridisierungsassays, Immunoassays, CRISPR-Cas-Assays, Molecular-Beacon-Assays, PCR, digitale PCR, qPCR und Echtzeit-PCR.

**12.** Verpackungsartikel, wobei der Verpackungsartikel ein Substrat umfasst, das gemäß dem Verfahren der Ansprüche 1-11 markiert wurde.

**13.** Kit zum Analysieren einer Bibliothek von Nukleinsäure-Sequenzmarkern, die gemäß dem Verfahren der Ansprüche 1-11 auf ein Substrat aufgebracht wurde, umfassend

Mittel zum Durchführen eines oder mehrerer der Analyseschritte gemäß dem Verfahren nach Anspruch 10 und mindestens eines der Verfahren gemäß Anspruch 11,
optional Mittel zur Nukleinsäureextraktion.

**14.** Nukleinsäure-Sequenzmarker-Bibliothek nach Anspruch 1 Schritt a. zur Verwendung in dem Verfahren der Ansprüche 1-11.

## Revendications

**1.** Procédé de marquage d’un substrat avec une bibliothèque unique d’étiquettes de séquences d’acides nucléiques comprenant :

a. la fourniture d’une bibliothèque d’étiquettes de séquences d’acides nucléiques, comprenant au moins deux groupes différents d’étiquettes de séquences d’acides nucléiques qui comprennent chacun une pluralité d’étiquettes de séquences d’acides nucléiques,

dans lequel chacune desdites étiquettes de séquences d’acides nucléiques comprend un motif d’acide nucléique défini dans au moins une première et une seconde position caractéristique, et dans lequel en dehors desdites au moins deux positions caractéristiques, chaque étiquette de séquence d’acide nucléique comprend une séquence d’acide nucléique aléatoire,
dans lequel la combinaison des motifs d’acide nucléique de l’au moins une première position caractéristique et de l’au moins une seconde position caractéristique d’une étiquette de séquence d’acide nucléique est caractéristique et unique pour le groupe de ladite étiquette de séquence d’acide nucléique, et

b. le marquage du substrat avec la bibliothèque unique d’étiquettes de séquence d’acide nucléique en appliquant la bibliothèque d’étiquettes de séquence d’acide nucléique au substrat.

**2.** Procédé selon la revendication 1, dans lequel l’intégralité des étiquettes de séquence d’acide nucléique comprises dans la bibliothèque comprend dans chaque position de séquence d’acide nucléique chaque type de base d’acide nucléique qui est compris dans la bibliothèque à un rapport +/- égal à une marge d’erreur par rapport au nombre total de fragments dans l’intégralité de la bibliothèque et à la longueur de la séquence aléatoire de chaque étiquette de séquence d’acide nucléique.

**3.** Procédé selon l’une quelconque des revendications précédentes, dans lequel seules des combinaisons spécifiques de motifs d'acide nucléique dans l'au moins une première et l'au moins une seconde position caractéristique sont utilisées dans les étiquettes de séquence d'acide nucléique.

**4.** Procédé selon l'une ou l'autre des revendications précédentes, dans lequel,
si chaque position caractéristique comprend un motif d'une longueur d'un seul nucléotide, chaque type de base d'acide nucléique, compris dans la bibliothèque, est présent en proportion égale dans chacune de l'au moins une première et l'au moins une seconde position caractéristique de l'intégralité des étiquettes de séquence d'acide nucléique comprise dans la bibliothèque, comme

a. les bases d'acide nucléique dans l'au moins une première et l'au moins une seconde position caractéristique des étiquettes de séquence d'acide nucléique sont sélectionnées en conséquence et/ou les groupes d'étiquettes de séquence d'acide nucléique sont compris dans la bibliothèque selon un rapport spécifique, ou
b. une quantité suffisante d'étiquettes de séquences d'acide nucléique supplémentaires comprend des nucléobases sélectionnées dans l'au moins une première et l'au moins une seconde position caractéristique est comprise dans la bibliothèque.

**5.** Procédé selon l'une quelconque des revendications 1 à 3, dans lequel le motif d'acide nucléique dans l'au moins une première et l'au moins une seconde position caractéristique comprennent chacune une séquence nucléotidique d'au moins deux nucléotides consécutifs.

**6.** Procédé selon l'une quelconque des revendications précédentes, dans lequel au moins 80 %, de préférence au moins 90 %, des molécules d'étiquette de séquence d'acide nucléique de la bibliothèque comprend une séquence d'acide nucléique unique dans l'intégralité de la bibliothèque.

**7.** Procédé selon l'une quelconque des revendications précédentes, dans lequel la quantité de bibliothèque appliquée au substrat comprend 10 à 10^15 fois, de préférence 100 à 10^14 fois, le nombre de molécules d'étiquette de séquence d'acide nucléique à analyser (A) à partir de la bibliothèque appliquée pour une certitude spécifique de validation correcte (V), dans lequel la certitude spécifique de validation correcte (V) est calculée selon la formule :

$$V = (1 - (R^A))*100,$$

dans lequel R est le rapport entre le nombre de combinaisons uniques de motifs d'acide nucléique à l'au moins une première et l'au moins une seconde position caractéristique comprises dans la bibliothèque et le nombre de combinaisons uniques possibles de motifs.

**8.** Procédé selon l'une quelconque des revendications précédentes, dans lequel chaque étiquette de séquence d'acide nucléique est présente sous la forme d'une molécule d'acide nucléique circulaire ou est comprise à l'intérieur d'une molécule d'acide nucléique circulaire.

**9.** Procédé selon l'une quelconque des revendications précédentes, dans lequel un substrat est choisi parmi le groupe composé de papier, de carton, de toile, de céramique, de coton, de laine, de cuir, de nylon, de plastique, de vinyle, de pierre, de métal, de verre, de liquide, de produit pharmaceutique, d'article d'emballage, de sculptures, de peintures, d'œuvres d'art, d'ordinateurs, de dispositifs périphériques informatiques, d'imprimantes, de microprocesseurs, de téléviseurs, de systèmes audio, de meubles, d'appareils électroménagers, d'antiquités, de vêtements, de sacs à main, de chaussures, de lunettes de soleil, d'appareils photo, d'objets imprimés en 3D, d'automobiles, de bicyclettes, de motocyclettes, de bagages ou d'objets de collection.

**10.** Procédé selon l'une quelconque des revendications précédentes, dans lequel la bibliothèque d'étiquettes de séquence d'acide nucléique appliquée au substrat est configurée pour être analysée

a. en extrayant les étiquettes de séquence d'acide nucléique de la bibliothèque à partir du substrat et en analysant les étiquettes de séquence d'acide nucléique, ou
b. en analysant les étiquettes de séquence d'acide nucléique de la bibliothèque directement sur le substrat.

**11.** Procédé selon la revendication 10, dans lequel l'analyse des étiquettes de séquence d'acide nucléique comprend la réalisation d'un ou de plusieurs des procédés d'analyse sélectionnés parmi le groupe comprenant le séquençage d'acide nucléique, les tests de restriction d'acide nucléique, les tests d'hybridation, les immunoessais, les tests CRISPR-Cas, les tests de balise moléculaire, le PCR, le PCR numérique, le qPCR et le PCR en temps réel.

**12.** Article d'emballage, dans lequel l'article d'emballage comprend un substrat qui a été marqué selon le procédé des revendications 1 à 11.

**13.** Kit pour l'analyse d'une bibliothèque d'étiquettes de séquence d'acide nucléique qui a été appliquée à un substrat selon le procédé des revendications 1 à 11, comprenant

- des moyens permettant d'effectuer une ou plusieurs des étapes d'analyse selon le procédé de la revendication 10 et au moins l'un des procédés selon la revendication 11,
- éventuellement des moyens pour l'extraction d'acide nucléique.

**14.** Bibliothèque d'étiquettes de séquence d'acide nucléique selon l'étape a. de la revendication 1, pour utilisation dans le procédé selon les revendications 1 à 11.

Fig. 1

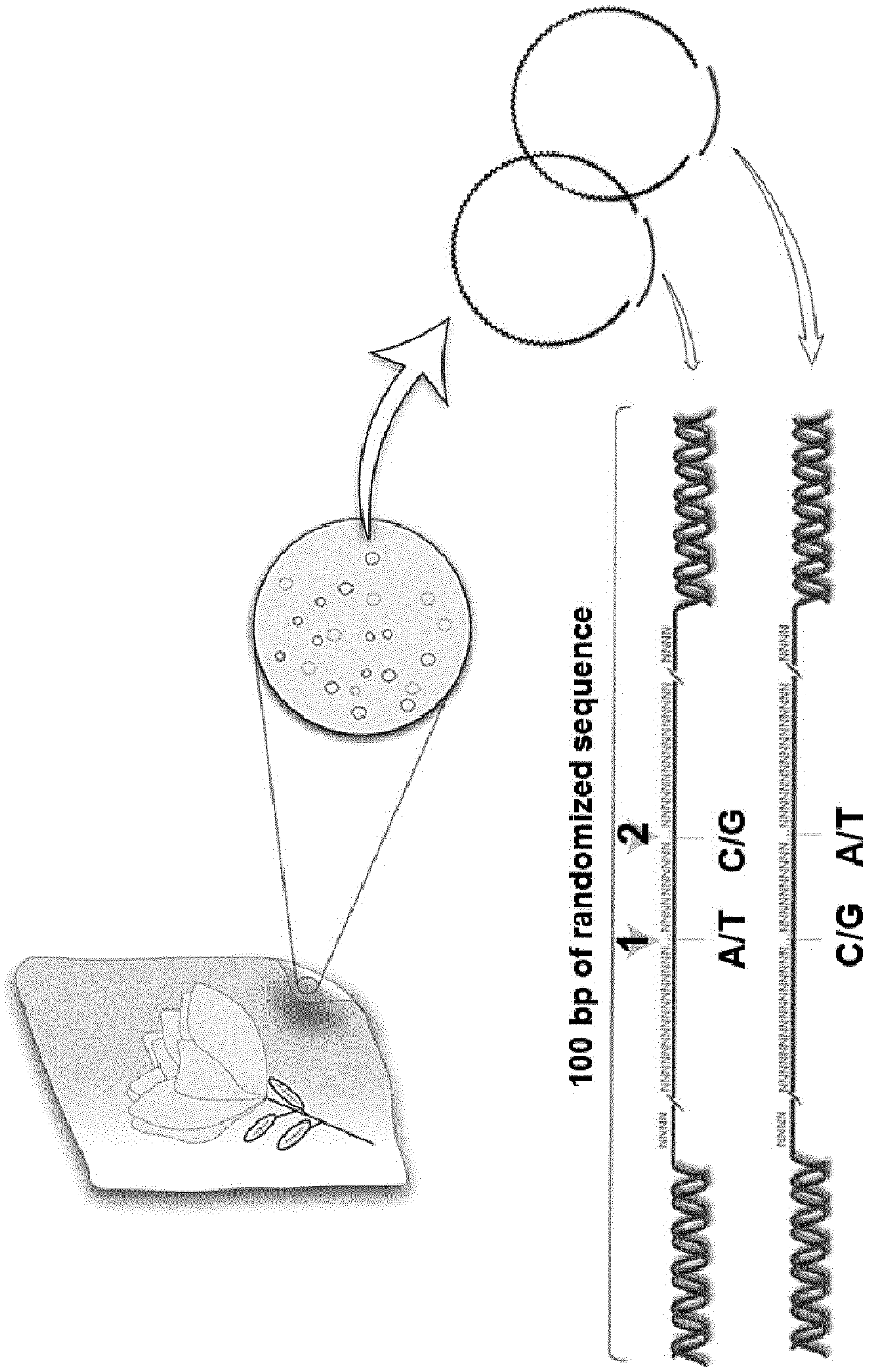

Fig. 2

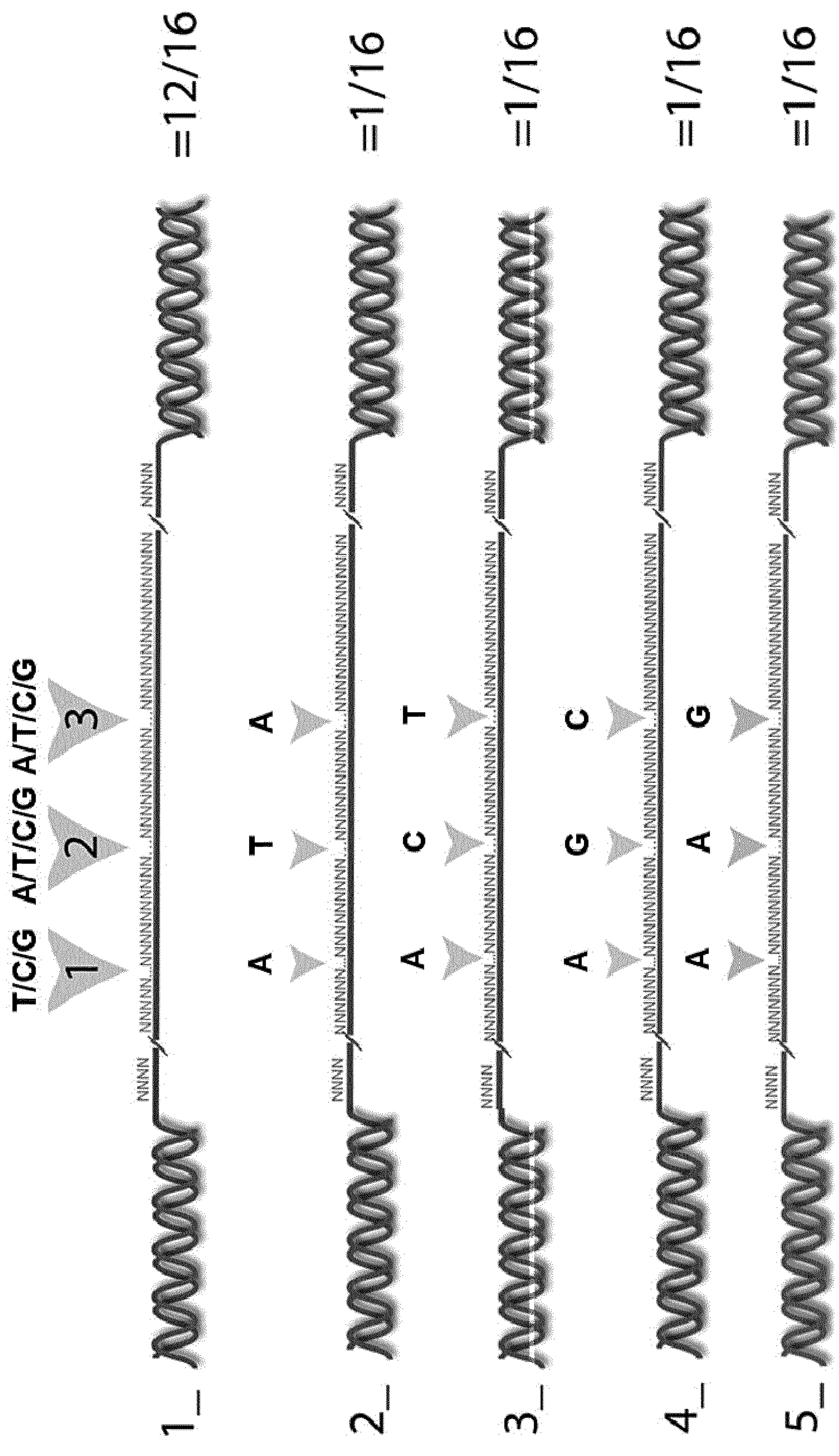

Fig. 3

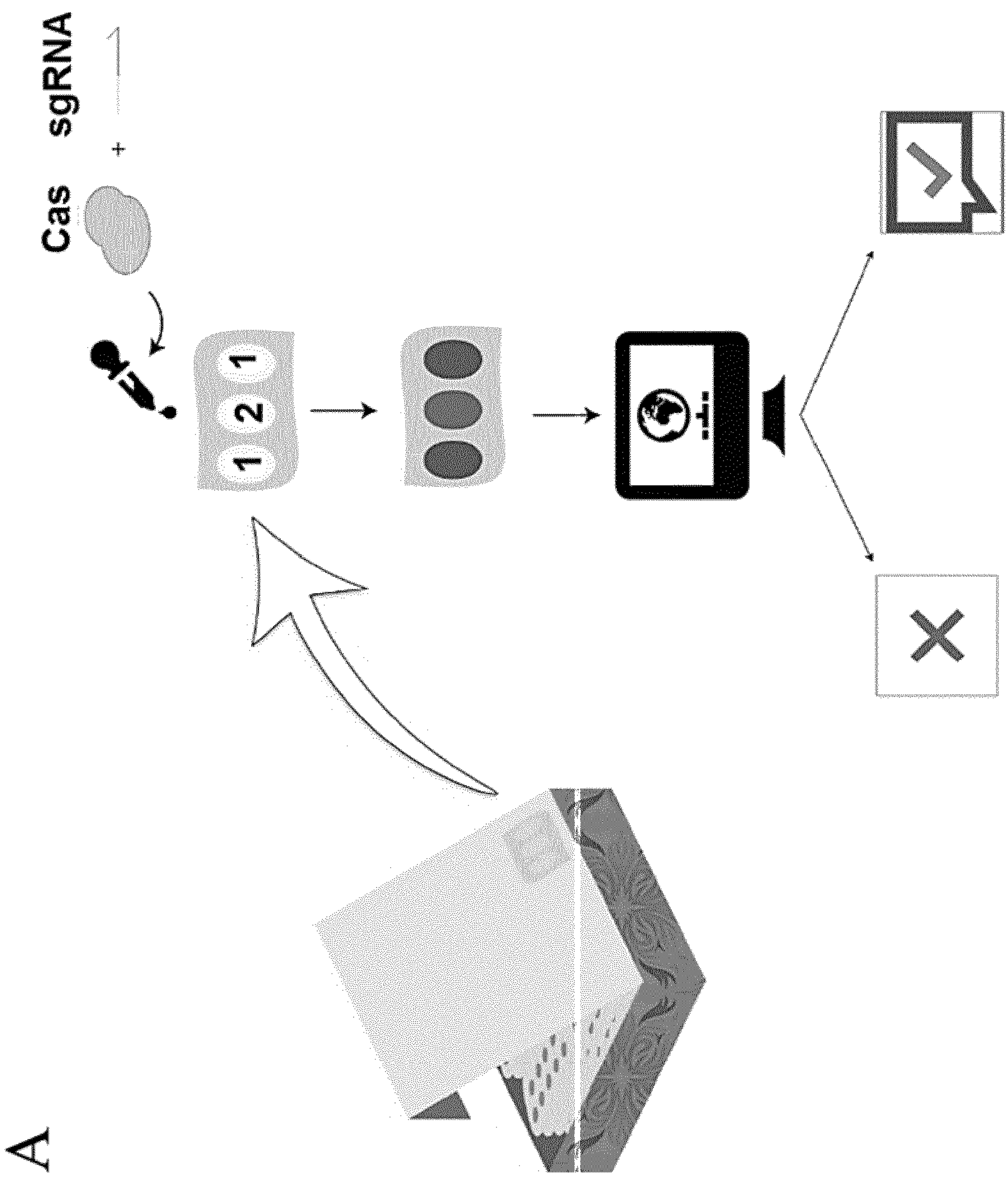

Fig 3. continued

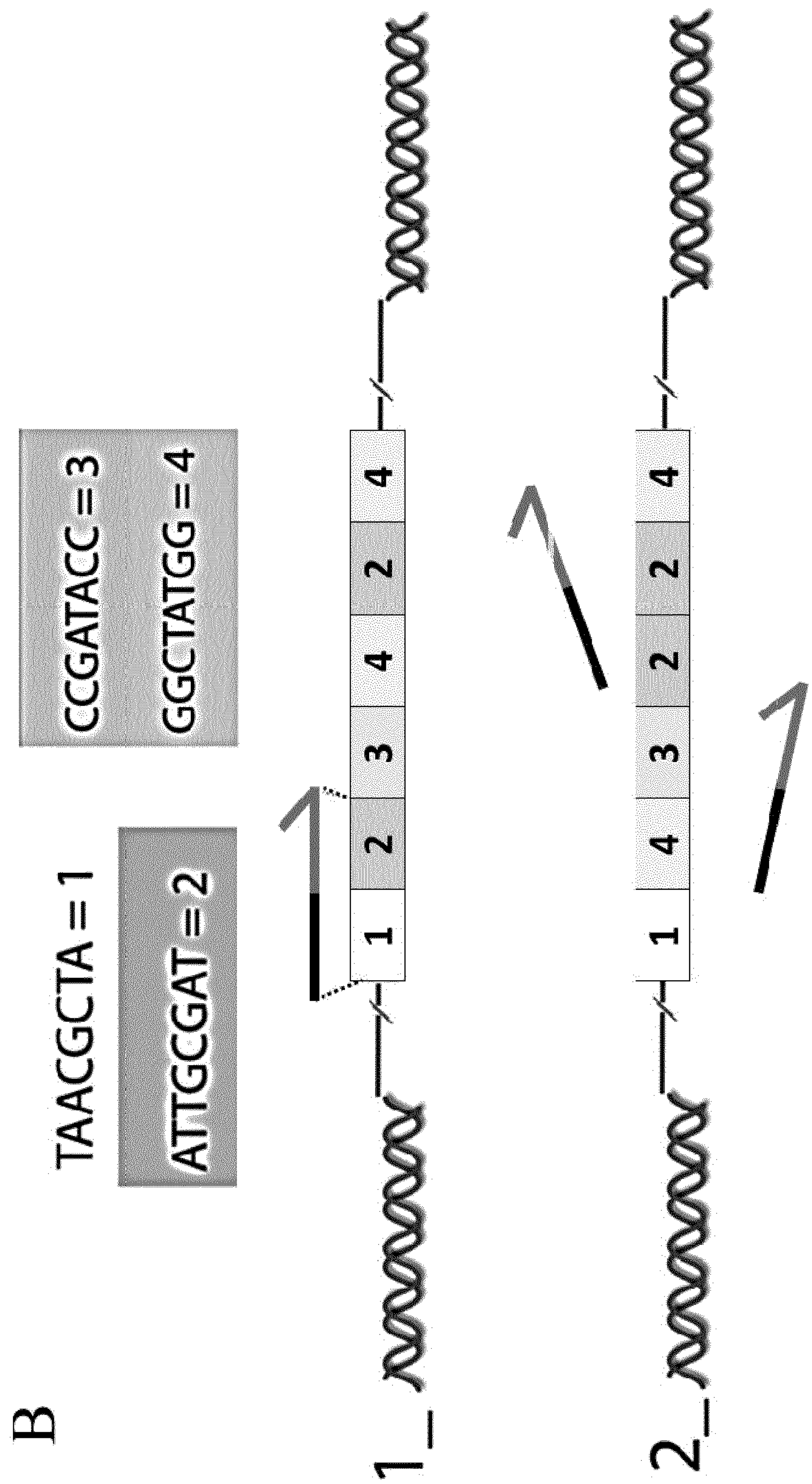

Fig. 4

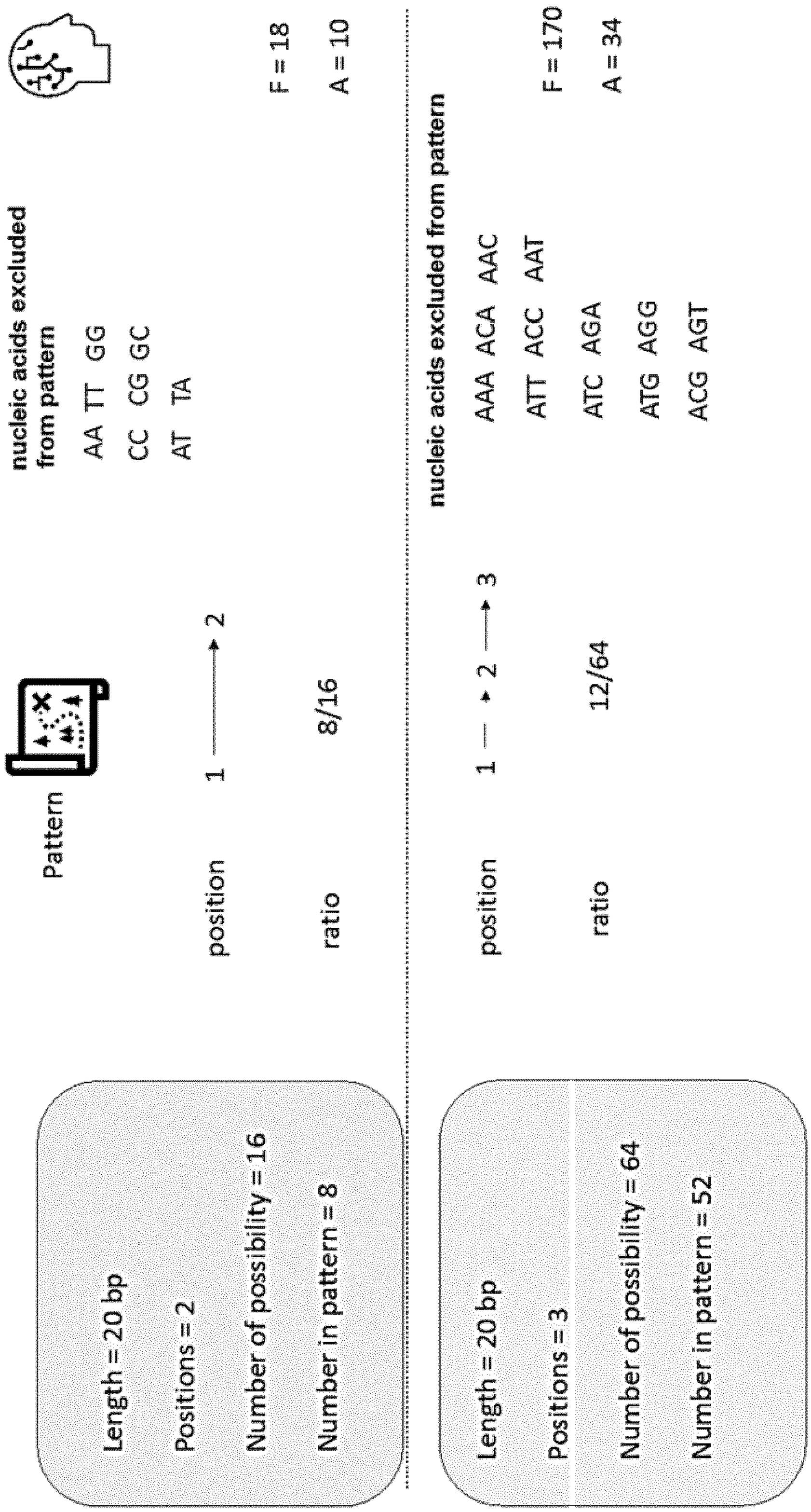
Length = 20 bp
Positions = 2
Number of possibility = 16
Number in pattern = 8
Pattern
position 1 → 2
ratio 8/16
nucleic acids excluded from pattern
AA TT GG
CC CG GC
AT TA
F = 18
A = 10
Length = 20 bp
Positions = 3
Number of possibility = 64
Number in pattern = 52
position 1 → 2 → 3
ratio 12/64
nucleic acids excluded from pattern
AAA ACA AAC
ATT ACC AAT
ATC AGA
ATG AGG
ACG AGT
F = 170
A = 34

Fig. 5

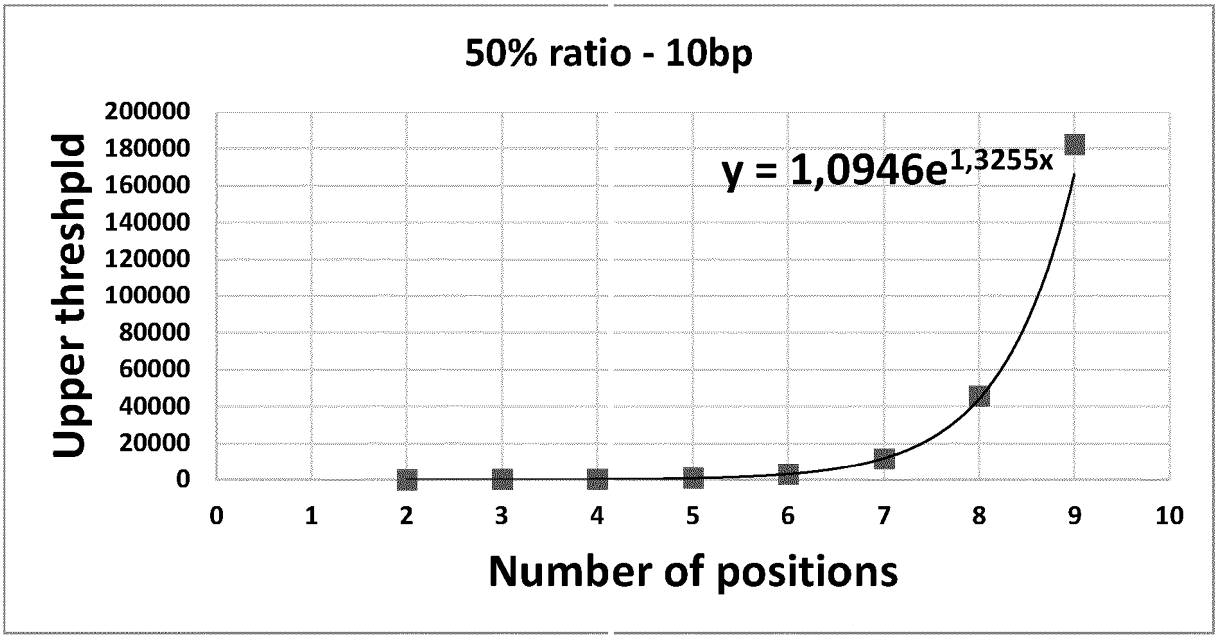
50% ratio - 10bp
Upper threshpld
200000
180000
160000
140000
120000
100000
80000
60000
40000
20000
0
0
1
2
3
4
5
6
7
8
9
10
Number of positions
y = 1,0946e1,3255x

Fig. 6

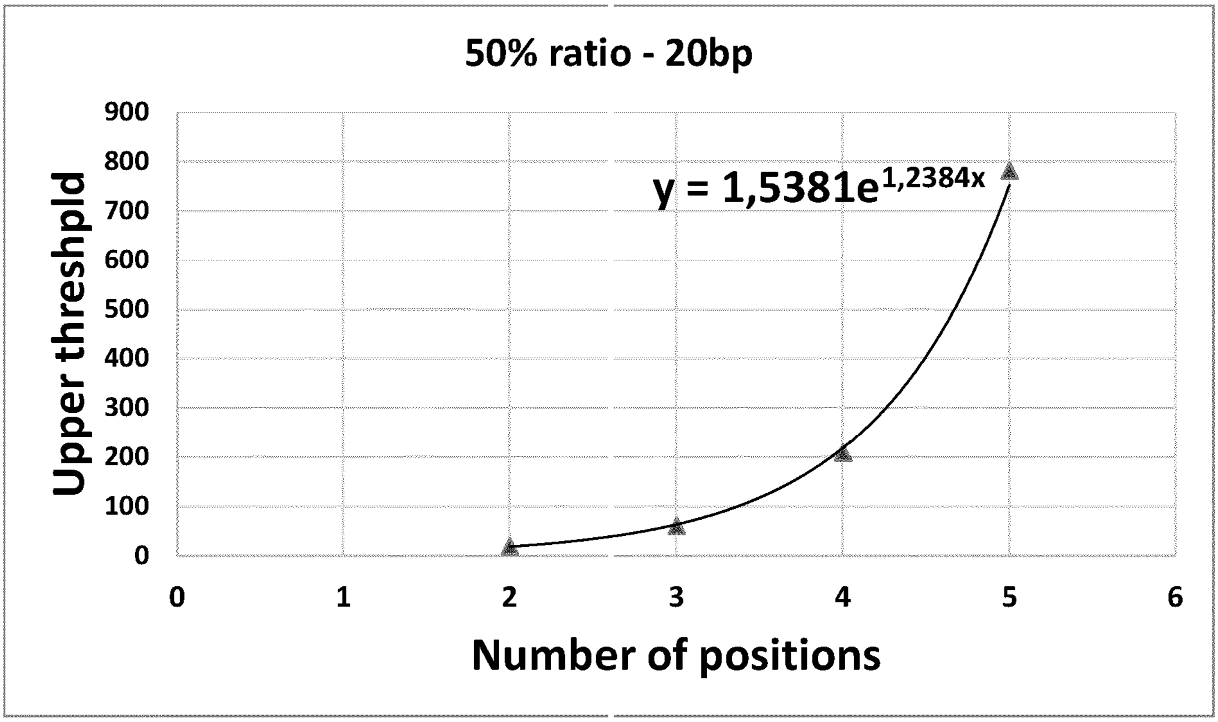
50% ratio - 20bp
y = 1,5381e^1,2384x
Upper threshpld
900
800
700
600
500
400
300
200
100
0
0
1
2
3
4
5
6
Number of positions

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019236787 A1
- US 20050059059 A1
- US 20210108194 A1


### Non-patent literature cited in the description


- **DOROSCHAK et al.** *Nat Commun*, 2020
- **ITAKURA et al.** *Int J Inf Secur*, 2002
- **HASHIYADA et al.** *J Exp Med*, 2004
- **FAN et al.** *Nat Biotechnol*, 2021
- **KELLNER**. *Nat Protoc*, 2019
- **CHEN et al.** *Science*, 27 April 2018, vol. 360 (6387), 436-439
- **CHEN et al.** *Science*, 2018
- **KELLNER et al.** *Nature Protocols*, 2019
- **MCCOWN et al.** *WIREs RNA*, 2020
- **SOOD et al.** *J Cheminform*, 2019, https://dnamod.hoffmanlab.org
- **PAK et al.** *Adv. Sci*, 2021
- **MUSTAFA et al.** *J. Clin. Microbiol.*, 2021
- **BAXEVANIS et al.** *Bioinformatics*, 2004
- **KELLNER et al.** *Nature protocols*, 2019